# EUROPEAN PATENT APPLICATION

(11) **EP 2 360 277 A1**
(43) Date of publication of application: **24.08.2011**
(21) Application number: 10188343.7
(22) Date of filing: 03.05.2007
(51) Int. Cl.: C12Q 1/68, G01N 33/53

(54) **Methods for diagnosing and predicting non-alcoholic steatohepatitis (NASH)**

(30) Priority: 03.05.2006 US 797457 P
(62) Divisional of application: 07776794.5
(71) Applicant: Geisinger Clinic, Danville, PA 17822-3021 (US)
(72) Inventor: Gerhard, Glenn S., Lewisburg, PA 17837 (US); Still, Christopher Dubet, Winfield, PA 17889 (US)
(74) Representative: Dörries, Hans Ulrich

(57) **Abstract**

The invention provides a panel of genes useful for diagnosing non-alcoholic steatohepatitis (NASH). The invention also provides a method of diagnosing NASH in non-invasive assays based on the expression of particular genes in a panel of NASH-related genes. Methods of treatment for NASH and compositions for treating NASH are also provided.

## Description

### CROSS-REFERENCE TO RELATED APPLICATIONS

This Application claims benefit of U.S. Provisional Application No. 60/797,457, filed May 3, 2006, the disclosure of which is hereby incorporated by reference in its entirety.

### FIELD OF THE INVENTION

The invention relates to non-alcoholic steatohepatitis (NASH). More specifically, the invention relates to methods for assessing NASH in patients.

### BACKGROUND OF THE INVENTION

The obesity epidemic is fueling increases in a variety of disorders, including diabetes, cardiovascular disease, and hypertension. An important but understudied complication of obesity is non-alcoholic steatohepatitis (NASH). NASH is an often clinically undiagnosed liver disorder that can progress to cirrhosis, one of the top 10 causes of death in the United States and a premalignant condition for developing hepatocellular carcinoma (Thomas, M.B. and A.X. Zhu (2005) J. Clin. Oncol. 23(13):2892-2899). NASH has become a leading cause of cryptogenic cirrhosis (Clark, J.M. and A.M. Diehl (2003) JAMA 289(22): p. 3000-3004), and may eventually surpass alcohol as a leading cause of cirrhosis. Liver histology assessed on biopsy is the only definitive and clinically accepted method to make the diagnosis of NASH, which is based upon findings that include steatosis (fat accumulation), inflammation, and fibrosis (Brunt, E.M. et al. (1999) Am. J. Gastroenterol. 94(9): p. 2467-2474). An estimated 20% to 30% of adults in the United States have excess fat accumulation in the liver (Neuschwander-Tetri, B.A. and S.H. Caldwell (2003) Hepatology, 37(5):1202-1209), with about 10% (or 2% to 3% of all adults) that meet current diagnostic criteria for NASH. The molecular basis for how NASH develops and progresses to cirrhosis is poorly understood, no serum diagnostic tests are available, and no effective pharmacological therapies exist. This represents a huge unmet medical need.

NASH refers to findings on liver biopsy in patients with steatohepatitis in the absence of significant alcohol consumption (Neuschwander-Tetri, B.A. and S.H. Caldwell (2003) Hepatology 37(5):1202-1209). It is encompassed by the term non-alcoholic fatty liver disease (NAFLD), usually defined as fat accumulation in the liver exceeding 5% to 10% by weight, but it is estimated practically as the percentage of hepatocytes containing lipid observed by light microscopy. As used herein, the term NASH refers to the spectrum of histological abnormalities defined by NAFLD that includes both simple steatosis and NASH in its more extreme forms. While some controversy exists over how NASH is best defined, including the necessity and character of specific findings such as the amount and types of fat (macrovesicular and microvesicular), lobular inflammation (acute and/or chronic), and fibrosis (zone 3 and portal), published histological criteria proposed by Brunt are generally used (Brunt, E.M. et al. (1999) Am. J. Gastroenterol. 94(9): p. 2467-2474). Cryptogenic cirrhosis appears to represent a late phase of NASH that has lost the typical necroinflammatory and steatotic features in most patients.

NASH may be the most common of all liver disorders (Neuschwander-Tetri, B.A. and S.H. Caldwell (2003) Hepatology 37(5):1202-1209). Steatosis is present in 60-70% of obese and 35% of lean patients, with steatohepeatitis and fibrosis in 20-25% of obese and 2.7% of lean patients. Up to 2-3% of obese patients with NASH may have cirrhosis. Upwards of 70-75% of type 2 diabetic patients have some form of NASH. A number of reports have addressed clinical predictors of NASH. Among these, age greater than 40 to 50 years, and the severity of obesity, diabetes, or hyperlipidemia (especially hypertriglyceridemia) are among the most reliable, although lacking in specificity. The role of female gender has also been studied in some series. Advanced NASH appears to be the relatively increased in women, supporting a role for female gender as a risk factor for progression. Abnormal liver enzyme levels detected at routine evaluation is a common method of identifying patients with NASH. Elevation of aspartate aminotransferase (AST), alanine aminotransferase (ALT), and an AST: ALT ratio greater than 1.5 have been studied as predictors. These factors support the likelihood of finding more advanced disease on the initial biopsy, but are neither sensitive nor specific. They may also carry long-term prognostic significance. However, clinicians well know that NASH is often found with liver enzymes in the normal range. It is also not uncommon for obese patients to harbor undiagnosed cirrhosis despite long-standing medical care because these patients often lack the classic nutritional changes of cirrhosis.

Liver biopsy remains the only means of assessing the presence and extent of specific necroinflammatory changes and fibrosis in NASH. However, firm recommendations of when to perform a liver biopsy in the routine clinical setting have not yet been developed. The use of surrogate markers, such as aminotransferases and fibrosis markers, are not adequate for monitoring disease as is necessary for clinical studies.

NASH is commonly associated with obesity, diabetes, and hyperlipidemia, as well as hypertension, hyperuricemia, and polycystic ovarian disease. NASH can be found in patients with lipodystrophies (Garg, A. (2000) Am. J. Med. 108(2):143-152), in addition to a variety of other disorders such as peroxisomal diseases, mitochondrialopathies, Weber-Christian disease, Wilson's Disease, industrial solvent exposure, medications (amiodarone, tamoxifen, nucleoside analogues, and methotrexate), celiac disease, and abetalipoproteinemia (Neuschwander-Tetri, B.A. and S.H. Caldwell (2003) Hepatology 37(5):1202-1209). A common feature in these disorders is abnormal fat metabolism and/or mitochondrial injury or dysfunction.

The 5- and 10-year survival in NASH has been estimated at 67% and 59%, respectively, although death often may be from co-morbid conditions (Propst, A. et al. (1995) Dig. Dis. Sci. 40(8):1805-1815). Familial associations have also has been described (Struben, V.M. et al. (2000) Am. J. Med. 108(1):9-13), suggesting that genetic factors may predispose to the development of NASH.

The development of steatosis, steatohepatitis, progressive hepatic fibrosis, and cirrhosis is most likely the result of multiple metabolic abnormalities. While a detailed and integrated pathophysiology that encompasses the primary findings in NASH has not yet emerged, a plausible paradigm for pathogenesis is the "two-hit" hypothesis (Day, C.P. and O.F. James (1998) Gastroenterology 114(4):842-845). The first "hit" is the accumulation of fat in hepatocytes, which may result from many conditions, including hyperlipidemia, diabetes mellitus, and obesity. Inhibition of the oxidation of fatty acids, increased delivery of fatty acids to the liver, and/or decreased synthesis or secretion of very-low-density lipoproteins may also play a role in the fat accumulation. The second "hit" is the trigger that leads to the characteristic inflammation, hepatocyte swelling and cell death, and subsequent fibrosis.

One major theory holds that increased hepatic oxidative stress from increased insulin resistance, increased free fatty acid metabolism, increased activity of mitochondrial uncoupling proteins, increased activity of cytochrome P-450 2E1, increased iron stores, and/or decreased antioxidant activities activates hepatic stellate cells with the ensuing generation of proinflammatory cytokines and progressive inflammation (Agrawal, S. and H.L. Bonkovsky (2002) J. Clin. Gastroenterol. 35(3):253-261).

Insulin resistance and hyperinsulinemia are primary abnormalities in NASH (Neuschwander-Tetri, B.A. and S.H. Caldwell (2003) Hepatology 37(5):1202-1209; Sanyal, A.J. (2001) Am. J. Gastroenterol. 96(2):274-276). A major mechanism of insulin resistance may be the downregulation of insulin receptor substrate 1 (IRS-1) signaling by excess free fatty acids. Fatty acids impair the tyrosine phosphorylation of IRS-1. Insulin sensitivity is also regulated by peptide mediators. Adipose tissue, especially mesenteric fat with its venous blood flowing directly to the liver, is a rich source of cytokine and peptide hormone production that regulates downstream metabolic activity.

Oxidative stress and mitochondrial dysfunction has been implicated as a central mechanism of hepatocellular injury in NASH (Caldwell, S.H. et al. (2004) Clin. Liver Dis. 8(3):595-617). Correlations between markers of oxidative stress and NASH have been made in animal models of fatty liver disease (Koteish, A. and A. Mae Diehl (2002) Best Pract. Res. Clin. Gastroenterol. 16(5):679-690). Multiple possible sources of oxidative stress in the fatty liver may play a role including cytochrome P450 peroxisomal beta oxidation, mitochondrial electron leak, and recruited inflammatory cells.

Increased levels of free fatty acids can be directly toxic to hepatocytes, as well as mediating insulin resistance (Neuschwander-Tetri, B.A. and S.H. Caldwell (2003) Hepatology 37(5):1202-1209). One mechanism governing the level of circulating lipids is the metabolism of fat stores in the periphery. The lipodystrophies (Garg, A. (2000) Am. J. Med. 108(2):143-152), genetic disorders characterized by the partial or complete inability to form adipose tissue, are associated with hepatic steatosis, NASH, and cirrhosis (Garg, A. and A. Misra (2002) J. Clin. Endocrinol. Metab. 87(7):3019-3022). Congenital generalized lipodystrophy is characterized by nearly absent peripheral fat, severe hepatic steatosis, and a significant risk for cirrhosis.

Energy metabolism may also play a role in NASH. Adenosine triphosphate (ATP) is necessary for maintaining cellular integrity, thus its depletion may predispose to hepatocellular injury. Hepatic ATP levels are depleted in NASH (Cortez-Pinto, H. et al. (1999) JAMA 282(17):1659-1664). Mitochondrial injury may be one cause of reduced hepatocellular ATP stores in NASH (Caldwell, S.H. et al. (2004) Clin. Liver Dis. 8(3):595-617). Electron microscopy studies have identified crystalline structures of uncertain composition with the mitochondrial matrix (Caldwell, S.H. et al. (1999) J. Hepato/. 31(3):430-434). Mitochondrial injury can lead to mutation and loss of mitochondrial DNA. Mitochondrial DNA damage or loss occurs in alcohol-induced liver injury (Mansouri, A. et al. (1997) J. Hepatol. 27(1):96-102) and in aging (Van Remmen, H. and A. Richardson (2001) Exp. Gerontol. 36(7):957-968). Limited data suggest that mitochondrial DNA damage also occurs in NASH (Caldwell, S.H. et al. (1999) J. Hepatol. 31(3):430-434).

Liver fibrosis results from chronic cellular injury that induces the deposition of extracellular matrix proteins such as collagens (Bataller, R. and D.A. Brenner (2005) J. Clin. Invest. 115(2):209-218). The main causes of liver fibrosis include chronic HCV infection, alcohol abuse, and NASH. The accumulation of extracellular matrix proteins disrupts the histological integrity of the liver by forming a fibrous scar. The initial distribution of this fibrous material depends on the cause of the liver injury. In chronic viral hepatitis, the fibrosis tissue is initially located around portal tracts, while in alcohol-induced liver disease and NASH, it is located in pericentral and perisinusoidal areas. Fibrotic liver disease apparently progresses from collagen bands to bridging fibrosis to frank cirrhosis. The designation of cirrhosis occurs when nodules of regenerating hepatocytes develop.

Cirrhosis causes increased intrahepatic resistance to blood flow and portal hypertension, ascites, renal failure, hepatic encephalopathy, and variceal bleeding. Patients with cirrhosis can be relatively asymptomatic (compensated cirrhosis) for long periods prior to the onset of morbidity. Cirrhosis is associated with most cases of hepatocellular carcinoma (HCC). Approximately 18,000 new cases of HCC are projected to occur in the United States in 2005 (Thomas, M.B. and A.X. Zhu (2005) J. Clin. Oncol. 23(13):2892-2899). The incidence of HCC has doubled over the past 30 years and has continued to rise because of the large number of individuals infected with hepatitis C and the decades long interval between the initial infection and the development of HCC. NASH related cirrhosis is also increasing in the United States and may contribute significantly to a burgeoning HCC epidemic. HCC develops primarily in the context of liver cell injury, which leads to inflammation, hepatocyte regeneration, liver matrix remodeling, fibrosis, and ultimately, cirrhosis.

Histological examination of liver biopsy tissue is the gold-standard technique for the assessment of liver fibrosis (Neuschwander-Tetri, B.A. and S.H. Caldwell (2003) Hepatology 37(5):1202-1209; Zafrani, E.S., (2004) Virchows Arch. 444(1):3-12). However, liver biopsy is an invasive and expensive procedure, that is associated with major complications in about 0.5% of patients, including mortality (Thampanitchawong, P. and T. Piratvisuth (1999) World J. Gastroenterol. 5(4): p. 301-304). Sampling error can occur, especially when needle biopsies are obtained (Regev, A. et al. (2002) Am. J. Gastroenterol. 97(10):2614-2618). Reliable, simple, and noninvasive methods for assessing liver fibrosis are thus needed. Serum levels of proteins directly related to the hepatic fibrogenic process have been used as surrogate markers of liver fibrosis such as N-terminal propeptide of type III collagen, hyaluronic acid, and tissue inhibitor of metalloproteinase type 1 (Bataller, R. and D.A. Brenner (2005) J. Clin. Invest. 115(2):209-218). These markers may be useful for distinguishing cirrhosis in certain patients, but lack sufficient discriminating ability among stages of fibrosis. They are also not specific if fibrosis is present in other organs. Evaluation of potential therapeutic agents in clinical trials is therefore problematic due to the need for liver biopsies to assess liver fibrosis. Noninvasive biomarkers to assess liver fibrosis should facilitate the design of clinical trials.

Most studies on hepatic fibrosis to date have focused upon cirrhosis due to viral hepatitis or alcoholic liver disease using microarrays with a limited gene repertoire. The sources of tissue have also been from patients undergoing liver transplantation or from needle biopsies for diagnosis, thus from patients with relatively advanced stages of hepatic fibrosis.

Limited gene expression profiling has been reported in NASH. In one study (Younossi, Z.M. et al. (2005) Liver Int. 25(4):760-771), a group of 29 NASH patients (12 with steatosis alone, the remainder unspecified), seven obese controls, and six non-obese controls were analyzed using customized cDNA microarrays containing 5220 genes. Only 8 genes showed significant differential expression in patients with NASH compared to obese controls. In another study, gene expression in liver from patients with cirrhosis due to NASH was compared with cirrhosis secondary to hepatitis C virus (HCV) infection, patients with cirrhosis secondary to primary biliary cirrhosis and healthy controls (Sreekumar, R. et al. (2003) Hepatology 38(1):244-251). The total number of subjects in each group was 6 and the oligonucleotide Affymetrix Hu6800 array containing probes for 6,412 known genes was used. The mean age of the 4 groups was approximately 50 and the mean BMI about 30, with 4 female and 2 male Caucasian subjects in each group. Of 6,412 genes surveyed, only 16 were expressed differentially in subjects with NASH when compared with all of the other groups (NASH vs. healthy controls, NASH vs. HCV, NASH vs. PBC). Twelve genes were underexpressed significantly among subjects with NASH.

More studies have been reported in hepatic fibrosis/cirrhosis from causes other than NASH, especially hepatitis viruses. For example, in a study from Japan, an oligonucleotide microarray of 3,800 genes was used to analyze RNA from fibrotic liver biopsies from 19 patients with hepatitis C virus (HCV) infection, including 8 with hepatocellular carcinoma, and 3 patients with metastatic cancer involving the liver (Shao, R.X. et al. (2005) World J. Gastroenterol. 11 (13):1995-1999). In the HCV-infected livers, genes involved in immune responses were highly expressed. In a comparison of bridging fibrosis and cirrhosis versus less severe fibrosis, expression levels of genes for plasma proteins (including albumin and apolipoprotein) and drug-metabolizing enzymes (aldoketoreductase and cytochrome P450 genes) were decreased and those of genes involved in the cell cycle (*e.g.*, serine/threonine kinases) were increased. With low numbers of patients, potential variation from the small size of needle biopsies, and limited numbers of genes on the microarray, discrimination between severe and mild fibrosis could not be achieved.

In a larger study, liver tissue from 59 patients with cirrhosis due to hepatitis B (n=7) and HCV (n=11) infection, hereditary hemochromatosis (n=3), Wilson's disease (n=5), alcoholic liver disease (n=10), primary biliary cirrhosis (n=16), and autoimmune hepatitis (n=7) were studied with an 8,400 gene microarray (Kim, J.W. et al. (2004) Hepatology 39(2):518-527). The patients were grouped by risk of developing hepatocellular carcinoma, with hepatitis, hemochromatosis, and Wilson's disease in the high risk group and the other causes in the low risk group. A group of 556 genes was found to be discriminative between the groups. Metallothionein 1B, metallothionein 1E, metallothionein 1L, ceruloplasmin, ATP-binding cassette A1, calcium channel beta 3 subunit, protein S , carboxypeptidase B2, antithrombin III, tissue factor inhibitor, midkine, complement component 6, complement component 9, and CD5 antigen-like were among the most significant discriminatory genes. Comparison with hepatocarcinoma gene expression data revealed a common set of 273 genes.

Another study on HCV infected cirrhotic livers was performed using a microarray of approximately 13,600 genes compared with normal liver (Anders, R.A. et al. (2003) Am. J. Pathol. 162(3):991-1000). In addition, normal liver samples were compared with each other to determine normal physiologic variation in gene expression. A group of genes was found to exhibit variable expression in normal liver including those associated with stress, acute-phase immune response, and hepatic stellate cell activation. These genes, as well as those associated with hepatocellular carcinoma were excluded were subtracted from the sets of genes differentially expressed in cirrhotic livers. The gene sets associated with HCV-infected cirrhotic liver genes expressed in activated lymphocytes and activated liver macrophages, genes involved in remodeling of extracellular matrix, genes related to the anti-apoptotic pathway of Bcl-2 signaling, and genes involved with the interferon response and virus-host interactions.

Human liver samples from seven male patients with advanced end-stage alcohol induced cirrhosis were compared with seven normal livers using a 3600 gene microarray (Seth, D., et al. (2003) Am. J. Pathol. 163(6):2303-2317). In parallel, alcohol-fed baboon liver was also analyzed. Genes related to fibrogenesis, xenobiotic metabolism, inflammation, oxidative stress, cell signaling were up-regulated. Collagens, matrix metalloproteinases, tissue inhibitors of matrix metalloproteinase, and annexin genes were up-regulated in humans.

A report from a Single Topic Conference held September 20-22, 2002, sponsored by the American Association for the Study of Liver Diseases, focused on fatty liver disorders (Neuschwander-Tetri, B.A. and S.H. Caldwell (2003) Hepatology 37(5):1202-1209). This conference discussed both clinical and basic aspects of NASH. Points relevant to this application include a lack of generally recognized indications for liver biopsy in NASH, and that liver enzymes are insensitive and cannot be used reliably to confirm the diagnosis of NASH nor stage the extent of fibrosis. The conference report concluded that: "The research agenda for the future includes establishing the role of insulin resistance and abnormal lipoprotein metabolism in NASH, determining the pathogenesis of cellular injury, defining predisposing genetic abnormalities, identifying better noninvasive predictors of disease, and defining effective therapy."

There is thus a great need in the art for a non-invasive diagnostic test for NASH and for targets for therapy for NASH.

### SUMMARY OF THE INVENTION

The invention provides a panel of genes associated with NASH which have high correlation with the onset and progression of NASH. As such, the invention provides a diagnostic tool comprising a panel of genes that are associated with NASH wherein the Panel comprises at least two genes selected from the genes shown in **Table 1.** In some embodiments, the panel comprises at least one small inducible cytokine. In some embodiments the small inducible cytokine is selected from the group consisting of CCL1 9, SCYA20, SCYA21, and CXCL6. In some embodiments, the panel comprises at least SCYA20 or at least CCL19. In one embodiment the panel is only CCL19. In another embodiment, the panel is only SCYA20.

The invention provides a method of diagnosing NASH by determining the expression levels of at least one of a panel of genes that are associated with NASH as compared to normal patients. The expression levels may be determined at the nucleic acid level or the amino acid level. In some embodiments, the expression level of a plurality of genes selected from **Table 1** is used. In some embodiments, the panel includes at least one of the small inducible cytokines (CCL19, SCYA20, SCYA21 and CXCL6). In some preferred embodiments, the expression of the genes in the panel is determined in a blood-based assay in which the expression level of the gene(s) is determined in a sample of blood taken from a patient. The expression level of the gene associated with NASH is compared to a normal level of expression of the gene. As shown herein, an elevated expression of some genes correlates with the incidence of NASH. In one embodiment, the expression level of CCL19 is compared with normal CCL19 expression and an increase in expression correlates with NASH. In another embodiment, the expression level of SCYA20 is compared with normal SCYA20 expression and an increase in expression correlates with NASH.

The invention also provides a method of treating NASH by modulating the expression or activity of genes associated with NASH. In some embodiments, the gene expression is increased. In other embodiments, the gene expression is decreased. In other embodiments, the level of expression is unmodified, but the activity of the protein product is altered due to modulation of the receptor for the protein or by disrupting intercellular signaling. In some embodiments, the expression level is decreased by the treatment. In some embodiments, the gene expression level of at least one gene in **Table 1** is decreased by the treatment. In some embodiments, the expression level of a plurality of genes selected from **Table 1** is decreased by the treatment. In some embodiments, the expression level of at least one of the small inducible cytokines (CCL19, SCYA20, SCYA21 and CXCL6) is decreased by the treatment. In some embodiments, the expression level of CCL19 is decreased. In some embodiments, the expression level of SCYA20 is decreased.

In the methods of treatment of the invention, the compounds that are used for treatment to inhibit the expression or biological activity of the proteins or their receptors are inhibitors that include, but are not limited to, antisense oligonucleotides, morpholino oligonucleotides, RNAi, antibodies, antibody fragments, or small molecules.

The invention also provides a method for discovering compounds that are useful in treating NASH or slowing the progression of NASH. The methods include contacting at least one protein from **Table 1** with a test substance and determine whether the test substance binds to the protein from **Table 1** or its receptor. Such assays may be performed *in vitro* and may be in a cell-based assay. Further, in the methods of the invention, a test substance may be determined to inhibit the biological activity of the protein or its receptor in cell based assays and thereby be identified as a compound useful for the treatment of NASH.

The invention also provides kits for diagnosis of NASH from patient samples (preferably blood) that may be nucleic acid based tests or protein-based tests. In some embodiments, the kit contain at least one polynucleotide that binds to a polynucleotide encoding a NASH-related protein. In some embodiments, the kit contains at least one antibody or antigen-binding portion of an antibody that specifically binds to at least one NASH-related protein. In some embodiments, the NASH-related protein is a small inducible cytokine. In other embodiments, the NASH related protein is at least one protein from **Table 1.**

### BRIEF DESCRIPTION OF THE DRAWINGS

**Figure 1** shows alkaline phosphatase, ALT, AST and BMI in patients with normal liver histology (white) NASH with no perisinusoidal fibrosis (light grey), and perisinusoidal fibrosis (dark grey). *p<0.01.

**Figure 2** shows serum HDL cholesterol level in patients with normal liver histology (n=30/15), NASH with no perisinusoidal fibrosis (n=66/20), and perisinusoidal fibrosis (n=26/9) who were not on statin therapy (white), or were on statin therapy (dark). *p<0.05.

**Figure 3** shows representative liver histology (100X) from patients. **Panel A:** normal appearing H&E stained section; **Panel B:** moderate steatosis and mild steatohepatitis but no fibrosis on H&E; **Panel C:** moderate steatosis and mild steatohepatitis and mild perisinusoidal fibrosis on H&E; **Panel D:** same as C but with trichrome stain for fibrosis; Panel E: bridging fibrosis on H&E; **Panel F:** same as E but with trichrome stain for fibrosis.

**Figure 4** shows hierarchical clustering diagram of gene expression profiling of pooled liver RNA (n=9/pool) using Affymetrix GeneChips. Normal, normal liver; Moderate inflammation, liver with moderate steatohepatitis but no fibrosis; Bridging fibrosis, liver with moderate to severe steatohepatitis and bridging fibrosis.

**Figure 5** shows reverse transcription (RT)-PCR of human *Foie Gras* (*flj12716l*) mRNA. Left: MW, molecular weight markers; NL, normal histology; ST, mild steatosis; FL, non-bridging fibrosis; BF, bridging fibrosis. Right: 18S rRNA (as a control for input RNA).

**Figure 6** shows RT-PCR of GAPDH, CCL19 and CXCL6 from individual patients with normal liver histology or bridging fibrosis.

**Figure 7** shows RT-PCR of RNA from peripheral blood mononuclear cells from five morbidly obese patients. Marker lanes flank the gel and GAPDH is used as a control. Bands corresponding to two of the cytokines (CCL19 and CXCL6) were detected. No evidence of expression of CCL20 was found.

**Figure 8** shows a standard curve for the CCYA20 (CCL20) ELISA assay.

**Figure 9** shows CCYA20 (CCL20) protein levels measured by ELISA in serum from patients with NASH.

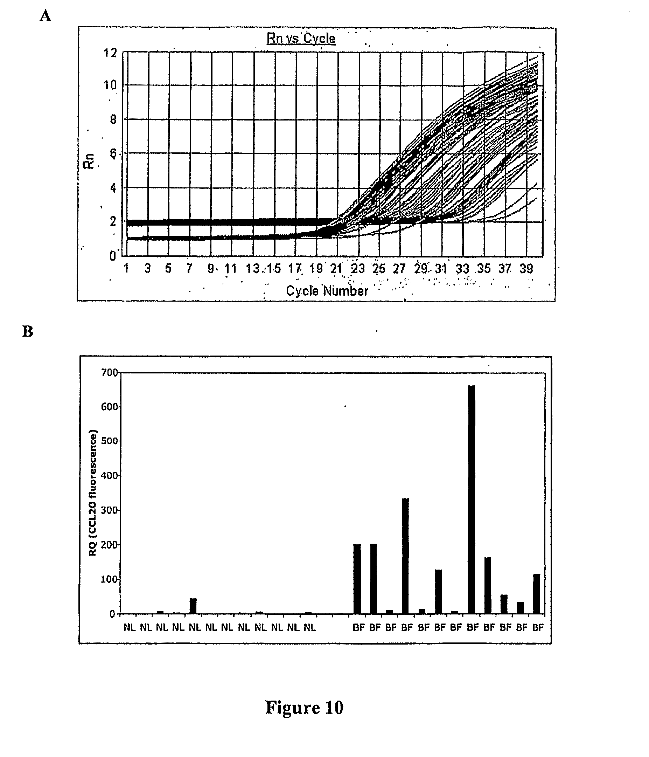

**Figure 10** shows real-time PCR of GAPDH and CCYA20 (CCL20); Panel A shows real-time PCR fluorescence plot of GAPDH (black lines) and CCL20 (grey lines) amplification of individual liver RNA samples from patients with normal histology and bridging fibrosis. The tight grouping of GAPDH fluorescence indicates that its expression level is similar across diverse samples and that the RNA preparations are of high quality; Panel B shows real-time PCR values obtained from experiment in A (NL=normal histology; BF= bridging fibrosis). Only one sample with normal histology was greater than the bridging fibrosis samples.

**Figure 11** shows real-time PCR fluorescence plot of GAPDH (black lines rising in later cycles) and CCL19 (grey lines rising in earlier cycles) amplification of individual PBMC RNA samples. The relatively tight grouping of many of the GAPDH fluorescence curves indicates that its expression level is similar across diverse samples and that the RNA is of very high quality. The CCL19 amplification plot indicated that levels were lower than those for GAPDH, and that a wider variation in levels was present.

### DETAILED DESCRIPTION OF ILLUSTRATIVE EMBODIMENTS

The reference works, patents, patent applications, and scientific literature, including accession numbers to GenBank database sequences that are referred to herein establish the knowledge of those with skill in the art and are hereby incorporated by reference in their entirety to the same extent as if each was specifically and individually indicated to be incorporated by reference. Any conflict between any reference cited herein and the specific teachings of this specification shall be resolved in favor of the latter.

Various definitions are made throughout this document. Most words have the meaning that would be attributed to those words by one skilled in the art. Words specifically defined either below or elsewhere in this document have the meaning provided in the context of the present invention as a whole and as are typically understood by those skilled in the art. Any conflict between an art-understood definition of a word or phrase and a definition of the word or phrase as specifically taught in this specification shall be resolved in favor of the latter. Headings used herein are for convenience and are not to be construed as limiting.

Standard reference works setting forth the general principles of recombinant DNA technology known to those of skill in the art include Ausubel et al., CURRENT PROTOCOLS IN MOLECULAR BIOLOGY, John Wiley & Sons, New York, 1998; Sambrook et al., MOLECULAR CLONING: A LABORATORY MANUAL, 2D ED., Cold Spring Harbor Laboratory Press, Plainview, New York, 1989; Kaufman et al., Eds., HANDBOOK OF MOLECULAR AND CELLULAR METHODS IN BIOLOGY AND MEDICINE, CRC Press, Boca Raton, 1995; McPherson, Ed., DIRECTED MUTAGENESIS: A PRACTICAL APPROACH, IRL Press, Oxford, 1991.

As used herein "NASH" refers to non-alcoholic steatohepatitis and without wishing to be bound by any particular theory of operability, it is believed that the genes discussed herein as associated with NASH are related to one or more components of NASH such as inflammation, fibrosis, steatosis and cirrhosis.

As used herein "isolated" means that the material is removed from its original environment (*e.g.*, the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

A "purified" or "substantially purified" polynucleotide or polypeptide is substantially separated from other cellular components that naturally accompany a native (or wild-type) nucleic acid or polypeptide and/or from other impurities (*e.g.*, agarose gel). A purified polypeptide or protein will comprise about 60% to more than 99% w/w of a sample, and may be about 90%, about 95%, or about 98% pure.

"About" as used herein refers to +/- 10% of the reference value.

As used herein, "variant" nucleotide or amino acid sequences refer to homologs, including, for example, isoforms, species variants, allelic variants, and fragments of the sequence of interest. "Homologous nucleotide sequence" or "homologous amino acid sequence," or variations thereof, refers to sequences characterized by a percentage identity (if referring to polynucleotides) or homology (if referring to polypeptides) of at least about 60%, at least about 70%, at least about 75%, at least about 80%, at least about 81%, at least about 82%, at least about 83%, at least about 84%, at least about 85%, preferably at least about 90%, at least about 95%, at least about 96%, at least about 97%, at least about 98%, or at least about 99%, and more preferably 100%, with respect to a reference sequence, or portion or fragment thereof encoding or having a functional domain. Homology refers to percentage identity of a reference amino acid sequence with allowance for conservative or non-conservative amino acid substitutions or both conservative and non-conservative amino acid substitutions.

The term "therapeutically effective amount" of a nucleic acid or protein associated with NASH, or antibody against a target for modulation or amelioration of NASH, means an amount calculated to achieve and maintain a therapeutically effective level in the liver, or in the plasma (if administered systematically), so as to inhibit NASH. Of course, the therapeutic dose will vary with the potency of each agonist or antagonist, and the rate of elimination or metabolism of the agonist or antagonist by the body and/or in the plasma. The therapeutic dose also applies to an amount of agonist or antagonist that would enhance the efficacy of any combined regimen of therapy.

As used herein, an "analyte" is a substance to be detected. It may be a nucleic acid (*e.g.*, DNA or RNA or fragment thereof), protein, protein fragment, peptide, metabolite, and the like.

As described in the Examples, several genes have been discovered to be associated with NASH through gene profiling. The genes that are useful for the method of the invention include, but are not limited to small inducible cytokines, collagens, matrix G1a protein, IL-8, EGF-containing fibulin-like extracellular matrix protein 1 (*e.g.*, those genes shown in **Table 1).**

Panels of genes may be selected to detect the presence of a NASH state in the body. The arrangement of genes in the Panel may be at least one of the genes in **Table 1,** for example, and any number of total genes selected from **Table 1,** such as 3, 4, 5, 6, 7, 8, 9 genes, etc., including the total number of the genes in **Table 1** and any subset of those genes, provided that if IL-8 is in the Panel, the Panel comprises at least one other gene from **Table 1.**

In some embodiments, the Panel comprises genes which are small inducible cytokines. For example, but not by way of limitation, the Panel comprises at least one small inducible cytokine selected from small inducible cytokine Subfamily A, members 19 (CCL19), 20 (SCYA20 or CCL20), 21 (SCYA21) and small inducible cytokine Subfamily B, member 6 (CXCL6)

In some embodiments the Panel comprises genes in the same family of genes, *e.g.*, small inducible cytokines, collagens, interleukins, matrix proteins, and the like. In other embodiments, the Panel comprises representative genes from more than one family of genes. In still other embodiments, the Panel comprises representatives from all families of genes.

In some embodiments, the Panel comprises at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or 36 genes from **Table 1.**

The Panel of genes of the invention may be used in a diagnostic screen for NASH by taking a sample from a patient and determining the level of expression of genes in the Panel and compare the results of the screen with a set of predetermined values for correlation of the results with likelihood of NASH in the patient.

In one embodiment of the method of the invention, a patient sample is contacted with a reagent to detect the presence of an analyte in the sample which corresponds to a gene or gene product associated with NASH. In some embodiments, the analyte is a nucleic acid corresponding to a gene associated with NASH and may be detected by any method known for nucleic acid detection and analysis, including, but not limited to microarray analysis (*e.g.*, using Affymetrix GeneChip Expression), PCR, RT-PCR, nucleic acid hybridization, nucleic acid protection assays, restriction digestion profiling and the like.

In another embodiment of the invention, a patient sample is contacted with a reagent to detect the presence of an analyte in the sample which corresponds to a gene product associated with NASH. In some embodiments, the analyte is a protein, peptide or protein fragment corresponding to a gene associated with NASH and may be detected by any method known for protein detection and analysis, including, but not limited to immunoassay (including, but not limited to ELISA, Western blot, radioimmunoassay, fluorescence-based immunoassays and the like), FPLC analysis and the like.

Antibodies to NASH proteins can be generated which are useful in detection, characterization or isolation of the corresponding proteins, as well as for modifying protein activity. Antibodies are generated by standard techniques, using human or animal purified or recombinant receptor proteins or fragments thereof as the immunogen.

Monoclonal antibodies to NASH proteins may be obtained, for example, as described for other proteins by Esmon, et al. (1993) Methods Enzymol. 222:359-385. The antibodies can be labeled using standard techniques, such as radiolabeling, enzyme labeling, fluorescent labels such as fluorescein, gold particles, dyes, and other means for detection of the antibodies. For example, antibodies can be biotinylated with biotinamidocaproate N-hydroxysuccinimide ester using standard procedures. Antibody can be immobilized to a solid support for use in immunoassays, for example, AffiGel-10^{™}, nitrocellulose, or microtiter wells, or use in solution phase immunoassays. In one embodiment, proteins are measured using ELISA assays in microtiter plates coated with mAb as described in Example 4.

In many cases, there are reagents known in the art for detecting the presence of the genes or gene products of the genes in **Table 1** known in the art. The sequences of the genes are known and one of skill in the art could use any of the available oligonucleotide predicting programs to derive oligonucleotide sequences that would be useful as probes or primers to detect the presence of the genes. In addition antibodies against the proteins and protein fragments can easily be made using standard techniques knowing the gene sequences. Further any of the available antibodies in the art that bind to and detect the proteins of the panel may be used in the method of the invention.

The method of the invention is suitable for screening of peripheral blood (either detecting the presence of the expression of the gene of interest in serum or in PBMCs). In the former case, detection of protein may be performed using ELISAs or other specific, quantifiable, protein detection methods and has the advantage of detecting proteins that may be of liver origin and are now circulating in the periphery. Without wishing to be bound by any particular theory of operation, it is believed that SCYA20 (CCL20) is a protein expressed in the liver and released and its circulating level can be detected and correlated with NASH (see Example 7).

In other embodiments, gene expression at the nucleic acid level is detected in PBMCs by specific, quantifiable methods such as real-time PCR on RNA extracted from PBMCs. The Taqman assay is well-known in the art as a means to quantify gene expression for analysis beginning with an RNA substrate. Any type of specific, quantifiable analysis known in the art may be used to detect gene expression and compare the expression level with normal levels to correlate either increased or decreased expression (depending on the gene of interest) with the diagnosis of NASH.

The invention also provides kits for diagnosis of NASH from patient samples (preferably blood) that may be nucleic acid based tests or protein-based tests. In some embodiments, the kit contain at least one polynucleotide that binds to a polynucleotide encoding a NASH-related protein. In some embodiments, the kit contains, in separate containers, a plurality of probes to detect one or more polynucleotides encoding one or more NASH-related proteins. In some embodiments, the proteins are small inducible cytokines, including, but not limited to CCL19, SCYA20, SCYA21 and CXCL6. In other embodiments, the proteins are one or more of the proteins shown in **Table 1.** In other embodiments, the kit contains at least one antibody or antigen-binding portion of an antibody that specifically binds to at least one NASH-related protein. In some embodiments, the kit contains, in separate containers, a plurality of antibodies or antigen-binding portions of antibodies (or mixtures thereof) to detect one or more NASH-related proteins. In some embodiments, the NASH-related proteins are small inducible cytokines including, but not limited to CCL19, SCYA20, SCYA21 and CXCL6. In other embodiments, the NASH related proteins include at least one protein from **Table 1**. The kits may also include instructions for use.

The invention also provides a method of treating and/or preventing NASH in a patient by modulating the expression of genes associated with NASH or by altering the signaling pathways of NASH.

In the method of the invention, a patient who has NASH or is at risk for developing NASH as predicted by the method of the invention is identified, and this patient may be treated with a regimen to modulate the expression of at least one gene associated with NASH or to alter the biological activity of at least one expressed protein associated with NASH. In some cases, the modulation of the protein's biological activity could be accomplished by altering the expression or function of the receptor for the protein associated with NASH. In certain embodiments the genes or proteins that are modulated are those proteins shown in **Table 1** or receptors therefor. In some embodiments, the genes or proteins modulated are small inducible cytokines or receptors therefor. In other embodiments the modulated genes or proteins are selected from CCL19, SCYA20, SCYA21, CXCL6 and receptors therefor.

The manner of altering expression of genes associated with NASH could be by any means known in the art. For enhancing expression, one could introduce an expression vector into the cells of a patient either *in vivo* or *ex vivo* and increase the expression of a particular protein which correlates with NASH when its levels of expression are too low. Thus, by increasing expression of the protein, the progression or onset of NASH is ameliorated. Nucleic acids encoding two or more polypeptides may be incorporated into the same expression vector or may be present on separate expression vectors. Transfection of the vectors into cells may be simultaneous or sequential. Expression of the plurality of polypeptides may be under the control of the same regulatory elements or different regulatory elements to allow controlled expression of one or both polypeptides. Alternatively, the expression of the polypeptide(s) may be made to be constitutive.

Conversely, for genes which correlate with NASH when they are expressed at too high a level as compared to normal, various methods known in the art could be employed to reduce the expression or activity of the gene in such patients. By way of example, but not by limitation, one could alter expression of the gene using an inhibitor such as, for example, antisense oligonucleotides, morpholino oligonucleotides, inhibitory (RNAi), immunotherapy (either monoclonal antibodies or polyclonal antibodies or antibody fragments)

Antisense oligonucleotides may be used as therapeutic moieties in the treatment of disease states in animals and humans. Antisense oligonucleotide drugs, including ribozymes, have been safely and effectively administered to humans and numerous clinical trials are presently underway. It is thus established that oligonucleotides can be useful therapeutic modalities that can be configured to be useful in treatment regimes for treatment of cells, tissues and animals, especially humans

In the context of this invention, the term "oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases.

While antisense oligonucleotides are contemplated by the present invention, other oligomeric antisense compounds, including but not limited to oligonucleotide mimetics such as are described below, are also included. The antisense compounds in accordance with this invention preferably comprise from about 8 to about 50 nucleobases (*i.e*. from about 8 to about 50 linked nucleosides). In some embodiments, the antisense oligonucleotides comprise from about 8 to about 15 nucleobases. In other embodiments, the antisense oligonucleotides comprise from about 16 to about 25 nucleobases. In other embodiments, the antisense oligonucleotides comprise from about 20 to about 30 nucleobases. In other embodiments, the antisense oligonucleotides comprise from about 25 to about 35 nucleobases. In other embodiments, the antisense oligonucleotides comprise from about 30 to about 45 nucleobases. In other embodiments, the antisense oligonucleotides comprise from about 40 to about 50 nucleobases. Antisense compounds include ribozymes, external guide sequence (EGS) oligonucleotides (oligozymes), and other short catalytic RNAs or catalytic oligonucleotides which hybridize to the target nucleic acid and modulate its expression.

As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn the respective ends of this linear polymeric structure can be further joined to form a circular structure, however, open linear structures are generally preferred. Within the oligonucleotide structure, the phosphate groups are commonly referred to as forming the internucleoside backbone of the oligonucleotide. The normal linkage or backbone of RNA and DNA is a 3' to 5' phosphodiester linkage.

Specific examples of preferred antisense compounds useful in this invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

In some embodiments, the modified oligonucleotide backbones include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage *i.e.,* a single inverted nucleoside residue which may be a basic (the nucleobase is missing or has a hydroxyl group in place thereof). Various salts, mixed salts and free acid forms are also included.

In other preferred oligonucleotide mimetics, both the sugar and the internucleoside linkage, *i.e.,* the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos. 5,539,082; 5,714,331; and 5,719,262, each of which is herein incorporated by reference. Further teaching of PNA compounds can be found in Nielsen et al. (1991) Science 254:1497-1500.

Modified oligonucleotides may also contain one or more substituted sugar moieties.

A further modification includes Locked Nucleic Acids (LNAs) in which the 2'-hydroxyl group is linked to the 3' or 4' carbon atom of the sugar ring thereby forming a bicyclic sugar moiety. The linkage is preferably a methylene (-CH₂-)ₙ group bridging the 2' oxygen atom and the 4' carbon atom wherein n is 1 or 2. LNAs and preparation thereof are described in WO 98/39352 and WO 99/14226.

Other modifications include 2'-methoxy (2'-O-CH₃), 2'-aminopropoxy (2'-OCH₂CH₂CH₂ NH₂), 2'-allyl (2'-CH₂-CH=CH₂)_{,} 2'-O-allyl (2'-O-CH₂-CH=CH₂) and 2'-fluoro (2'-F). The 2'-modification may be in the arabino (up) position or ribo (down) position. A preferred 2'-arabino modification is 2'-F. Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C=C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-aminoadenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (*e.g.* 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deazaadenine, 7-deazaguanosine, 2-aminopyridine and 2-pyridone. Further nucleobases include those disclosed in U.S. Pat. No. 3,687,808, those disclosed in THE CONCISE ENCYCLOPEDIA OF POLYMER SCIENCE AND ENGINEERING, pages 858-859, Kroschwitz, J. I., ed. John Wiley & Sons, 1990, those disclosed by Englisch et al. Angewandte Chemie, International Edition, 1991, 30, 613, and those disclosed by Sanghvi, Y. S., Chapter 15, ANTISENSE RESEARCH AND APPLICATIONS, pages 289-302, Crooke, S. T. and Lebleu, B. ed., CRC Press, 1993. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2°C. (Sanghvi, Y. S., Crooke, S. T. and Lebleu, B., eds., ANTISENSE RESEARCH AND APPLICATIONS, CRC Press, Boca Raton, 1993, pp. 276-278) and are presently preferred base substitutions, even more particularly when combined with 2'-O-methoxyethyl sugar modifications.

Another modification of the oligonucleotides of the invention involves chemically linking to the oligonucleotide one or more moieties or conjugates which enhance the activity, cellular distribution or cellular uptake of the oligonucleotide. The compounds of the invention can include conjugate groups covalently bound to functional groups such as primary or secondary hydroxyl groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugates groups include cholesterols, lipids, phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve oligomer uptake, enhance oligomer resistance to degradation, and/or strengthen sequence-specific hybridization with RNA. Groups that enhance the pharmacokinetic properties, in the context of this invention, include groups that improve oligomer uptake, distribution, metabolism or excretion. Conjugate moieties include but are not limited to lipid moieties such as a cholesterol moiety (Letsinger et al. (1989) Proc. Natl. Acad. Sci. USA 86:6553-6556), cholic acid (Manoharan et al. (1994) Bioorg. Med. Chem. Let. 4:1053-1060), a thioether, *e.g.*, hexyl-S-tritylthiol (Manoharan et al. (1992) Ann. N. Y. Acad. Sci. 660:306-309; Manoharan et al. (1993) Bioorg. Med. Chem. Let. 3:2765-2770), a thiocholesterol (Oberhauser et al. (1992) Nucl. Acids Res. 20:533-538), an aliphatic chain, *e.g.*, dodecandiol or undecyl residues (Saison-Behmoaras et al. (1991) EMBO J. 10:1111-1118; Kabanov et al. (1990) FEBS Lett. 259:327-330; Svinarchuk et al. (1993) Biochimie 75:49-54), a phospholipid, *e.g.*, di-hexadecyl-rac-glycerol or triethyl-ammonium 1,2-di-O-hexadecyl-rac-glycero-3-H-phosphonate (Manoharan et al. (1995) Tetrahedron Lett. 36:3651-3654; Shea et al. (1990) Nucl. Acids Res. 18:3777-3783), a polyamine or a polyethylene glycol chain (Manoharan et al. (1995) Nucleosides & Nucleotides 14:969-973), or adamantane acetic acid (Manoharan et al. (1995) Tetrahedron Lett. 36:3651-3654), a palmityl moiety (Mishra et al. (1995) Biochim. Biophys. Acta 1264:229-237), or an octadecylamine or hexylamino-carbonyl-oxycholesterol moiety (Crooke et al. (1996) J. Pharmacol Exp. Ther. 277:923-937. Oligonucleotides of the invention may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic.

It is not necessary for all positions in a given compound to be uniformly modified, and in fact more than one of the aforementioned modifications may be incorporated in a single compound or even at a single nucleoside within an oligonucleotide. The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, *i.e*., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Cleavage of the RNA target can be routinely detected by gel electrophoresis and, if necessary, associated nucleic acid hybridization techniques known in the art.

Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers.

The antisense compounds used in accordance with this invention may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is well known to use similar techniques to prepare oligonucleotides such as the phosphorothioates and alkylated derivatives.

The antisense compounds of the invention are synthesized *in vitro* and do not include antisense compositions of biological origin, or genetic vector constructs designed to direct the *in vivo* synthesis of antisense molecules. The compounds of the invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption.

A particular form of antisense technology are morpholino oligonucleotides (Summerton, J. and D. Weller (1997) Antisense Nucl. Acid Drug Dev. 7:187-195; Nasevicius, A. and S.C. Ekker (2000) Nat. Genet. 26:216-220; Yan, Y-K. et al. (2002) Development 129:5065-5079). Morpholino oligonucleotides are nonionic DNA analogs with altered backbone linkages compared with DNA or RNA, but follow Watson-Crick base-pairing with complementary sequences. Typically, morpholinos are at least about 18-25 nucleobases in length, in some embodiments, the morpholinos are at least about 25-30 nucleobases in length, in still more embodiments, the morpholinos are at least about 30-35 nucleobases in length or more. The strengths of morpholinos as tools for investigating vertebrate development are well described in a recent review by Ekker S.C. (2000) Yeast 17:302-306, the disclosure of which is hereby incorporated by reference.

Morpholinos form RNA-morpholino hybrids that are not substrates for RNase H, and are not degraded. Morpholino oligomers unambiguously block gene expression in a sequence-specific manner.

The present invention provides morpholino-modified oligonucleotides targeting the 5'untranslated region of a member of the panel of genes associated with NASH, or a polynucleotide or a splice site of a gene associated with NASH. Such morpholino-modified oligonucleotides are effective in inhibiting the expression of one or more of these genes and interfere with the onset or progression of NASH in animals treated with these morpholino-modified oligonucleotides.

Morpholinos are highly non-polar. Thus, modified or unmodified morpholino oligos, may be administered in combination with any known delivery vehicle/vector that facilitates delivery of morpholino oligos into cells/tissues.

The nucleic acid constructs of the invention may be delivered by any means known in the art. In some embodiments, nucleic acid is delivered into a cell using *ex vivo* strategies. In other embodiments, nucleic acid is delivered into a cell using *in vivo* strategies.

In *ex vivo* gene therapy methods, the cells are removed from the host organism, such as a human, prior to experimental manipulation. These cells are then transfected with a nucleic acid *in vitro* using methods well known in the art. These genetically manipulated cells are then reintroduced into the host organism. Alternatively, *in vivo* gene therapy approaches do not require removal of the target cells from the host organism. Rather, the nucleic acid may be complexed with reagents, such as liposomes or retroviruses, and subsequently administered to target cells within the organism using known methods. See, *e.g.*, Morgan et al. (1987) Science 237:1476, 1987; Gerrard et al. (1993) Nat. Genet. 3:180.

Several different methods for transfecting cells can be used for either *ex vivo* or *in vivo* gene therapy approaches. Known transfection methods may be classified according to the agent used to deliver a select nucleic acid into the target cell. These transfection agents include virus dependent, lipid dependent, peptide dependent, and direct transfection ("naked DNA") approaches. Other approaches used for transfection include calcium co-precipitation and electroporation.

Viral approaches use a genetically engineered virus to infect a host cell, thereby "transfecting" the cell with an exogenous nucleic acid. Among known viral vectors are recombinant viruses, of which examples have been disclosed, including poxviruses, herpesviruses, adenoviruses, and retroviruses. Such recombinants can carry heterologous genes under the control of promoters or enhancer elements, and are able to cause their expression in vector-infected host cells. Recombinant viruses of the vaccinia and other types are reviewed by Mackett et al. (1994) J. Virol. 49:3; also see Kotani et al. (1994) Hum. Gene Ther 5:19.

Non-viral vectors, such as liposomes, may also be used as vehicles for nucleic acid delivery in gene therapy. In comparison to viral vectors, liposomes are safer, have higher capacity, are less toxic, can deliver a variety of nucleic acid-based molecules, and are relatively nonimmunogenic. See Felgner, P. L. and Ringold, G. M., (1989) Nature 337:387-388. Among these vectors, cationic liposomes are the most studied due to their effectiveness in mediating mammalian cell transfection *in vitro.* One technique, known as lipofection, uses a lipoplex made of a nucleic acid and a cationic lipid that facilitates transfection into cells. The lipid/nucleic acid complex fuses or otherwise disrupts the plasma or endosomal membranes and transfers the nucleic acid into cells. Lipofection is typically more efficient in introducing DNA into cells than calcium phosphate transfection methods. Chang et al. (1988) Focus 10:66.

One known protein dependent approach involves the use of polylysine mixed with a nucleic acid. The polysine/nucleic acid complex is then exposed to target cells for entry. See, *e.g.,* Verma and Somia (1997) Nature 389:239; Wolff et al. (1990) Science 247:1465.

"Naked DNA" transfection approaches involve methods where nucleic acids are administered directly *in vivo.* See U.S. Pat. No. 5,837,693 to German et al. Administration of the nucleic acid could be by injection into the interstitial space of tissues in organs, such as muscle or skin, introduction directly into the bloodstream, into desirable body cavities, or, alternatively, by inhalation. In these so called "naked DNA" approaches, the nucleic acid is injected or otherwise contacted with the animal without any adjuvants. It has been reported that injection of free ("naked") plasmid DNA directly into body tissues, such as skeletal muscle or skin, can lead to protein expression. See Ulmer et al. (1993) Science 259:1745-1749; Wang et al. (1993) Proc. Nat. Acad. Sci. USA 90:4157-4160; Raz et al. (1994) Proc. Nat. Acad. Sci. USA 91:9519-9523.

Electroporation is another transfection method. See U.S. Pat. No. 4,394,448 to Szoka, Jr. et al. and U.S. Pat. No. 4,619,794 to Hauser. The application of brief, high-voltage electric pulses to a variety of animal and plant cells leads to the formation of nanometer-sized pores in the plasma membrane. DNA can enter directly into the cell cytoplasm either through these small pores or as a consequence of the redistribution of membrane components that accompanies closure of the pores.

The NASH-associated nucleic acid molecules, polypeptides (including cell permeable modified versions of the protein), and NASH-associated antibodies (also referred to herein as "active ingredients") of the invention, and derivatives, fragments, analogs and homologs thereof, can be incorporated into pharmaceutical compositions in therapeutically effective amounts suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of REMINGTON'S PHARMACEUTICAL SCIENCES, a standard reference text in the field, which is incorporated herein by reference. Preferred examples of such carriers or diluents include, but are not limited to, water, saline, ringer's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g.*, intravenous, intradermal, subcutaneous, oral (*e.g.*, inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the active ingredients in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressured container or dispenser which contains a suitable propellant, *e.g.*, a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g.*, with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (see U.S. Pat. No. 5,328,470) or by stereotactic injection (see *e.g.,* Chen et al. (1994) Proc. Natl. Acad. Sci. USA 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.*, retroviral vectors, the pharmaceutical preparation can include one or more cells that produce the gene delivery system.

The pharmaceutical compositions can be included in a container, pack, or dispenser together with instructions for administration.

Another aspect of the present invention is directed to methods of identifying compounds that bind to the proteins or nucleic acid molecules encoding the proteins associated with onset and/or progression of NASH. In one embodiment, the method comprises contacting a protein associated with NASH or a polynucleotide encoding a protein associated with NASH with a test compound, and determining whether the test compound binds the protein or polynucleotide associated with NASH. In some embodiments the protein associated with NASH is at least one protein selected from the group of **Table 1**.

Binding can be determined by any binding assays known in the art, including but not limited to, gel-shift assays, Western blots, radiolabeled competition assay, phage-based expression cloning, co-fractionation by chromatography, co-precipitation, cross-linking, interaction trap/two-hybrid analysis, southwestern analysis, ELISA, and the like, which are described in, for example, CURRENT PROTOCOLS IN MOLECULAR BIOLOGY, 1999, John Wiley & Sons, NY. The polypeptide or polynucleotide employed in such a test may either be free in solution, attached to a solid support, or located intracellularly, or associated with a cell fraction. In one embodiment of the invention, high throughput screening ("HTS") for compounds having suitable binding affinity to this protein(s) is employed. Large numbers of test compounds may be exposed to immobilized one or more proteins associated with NASH. Bound protein is then detected by methods well known in the art.

Another method for identifying ligands of a target protein is described in Wieboldt et al., Anal. Chem., 69:1683-1691 (1997). This technique screens combinatorial libraries of 20-30 agents at a time in solution phase for binding to the target protein. Agents that specifically bind to the target protein which are retained on the filter are subsequently liberated from the target protein and analyzed by HPLC and pneumatically assisted electrospray (ion spray) ionization mass spectroscopy. This procedure selects library components with the greatest affinity for the target protein, and is particularly useful for small molecule libraries.

Other embodiments of the invention comprise using antibody-based competitive screening assays. In one embodiment, specific, neutralizing antibodies that bind a protein associated with NASH specifically compete with a test compound for binding to a protein associated with NASH. In this manner, the antibodies can be used to detect the presence of any peptide that shares one or more antigenic determinants with a protein associated with NASH. Such binding studies may use labeled antibodies and/or labeled test compounds. An examples of such a procedure can be found in, for example, Lin, A.H. et al. (1997) Antimicrobial Agents and Chemotherapy 41(10):2127-2131.

Another aspect of the present invention is directed to methods of identifying compounds that modulate (*i.e*., increase or decrease) the biological activity of a protein associated with NASH. Such methods comprise contacting at least one protein associated with NASH with a test compound, and determining whether the compound affects the biological activity of one or more proteins associated with NASH in a positive (agonist) or negative (antagonist) way as compared to the activity of the proteins associated with NASH in the absence of the test compound. For the genes on **Table 1**, the goal is to decrease expression of these genes as the increased expression of these genes is associated with a NASH phenotype.

In some embodiments, the compounds that modulate expression of a protein associated with NASH or biological activity may be identified in an *in vitro* cell assay in which a test compounds is incubated with a cell expressing a protein associated with NASH or having a polynucleotide encoding a protein associated with NASH and determining the effect the test compound has on expression or biological activity of the protein associated with NASH. Modulators of such polynucleotides, proteins or activities activity will be therapeutically useful in treatment NASH. Compounds identified as modulating expression or biological activity *in vitro* may be further tested *vivo* to confirm relevant and effective activity.

Test compounds contemplated by the invention include compounds from chemical libraries, including natural products and/or synthetic products from combinatorial chemical synthesis. Such compounds may include random peptides, oligonucleotides, or organic molecules.

The following examples are merely illustrative of the invention and are not to be construed as limiting the invention in any way.

### EXAMPLES

### Example 1

### A. Identification of Genes Associated with NASH

Patient samples and corresponding clinical data from an ongoing clinical research program in obesity in the Comprehensive Weight Management Clinic at Geisinger Clinic were used in the study.

### 1. Clinical research program in obesity

Essentially all patients who are enrolled in the Bariatric Surgery Program in the Comprehensive Weight Management Clinic at Geisinger are recruited into an IRB approved research program in obesity and NASH. The following inclusion and exclusion criteria are used.

### A. Inclusion Criteria:

Competent patients eligible for Gastric Bypass Surgery based on NIH criteria; Body Mass Index or BMI >40 with 2 co-morbid conditions including Sleep Apnea and Hypertension; BMI > 45 with no comorbid condition.

### B. Exclusion Criteria:

Patients with severe psychological contraindications; Patients not willing to be compliant with pre-post surgical recommendations; Age <21 or >60; Pregnant Women; Presence of associated neoplasm that is expected to be fatal within 5 years; Obvious inflammatory disorder (*e.g.* hepatitis, infection, connective tissue disease); History of NASH inducing conditions including lipodystrophies, peroxisomal diseases, mitochondrialopathies, Weber-Christian disease, Wilson's Disease, industrial solvent exposure, medications (amiodarone, tamoxifen, nucleoside analogues, and methotrexate), celiac disease, and abetalipoproteinemia. History of viral hepatitis or alcoholism (tests to rule out viral or metabolic causes of fibrosis are subsequently performed on patients with fibrosis on liver biopsy).

Patients enrolled in the bariatric surgery program undergo a 6-8 month pre-operative assessment and preparation period. Initial consultation with physician staff includes comprehensive medical history and physical exam, individualized diet instruction, review of behavior modification modules, metabolic rate determination via MedGem, and physical activity prescription. Disease specific, standard of care laboratory tests are obtained including triglycerides, total cholesterol, HDL, LDL, hemoglobin A1C, insulin, glucose, albumin, total bilirubin, direct bilirubin, alkaline phosphatase, AST, ALT, total protein, TSH, Vit D, zinc, iron, TIBC, ferritin, PTH, folate, cortisol, TSH, and testosterone/estrogen/progesterone levels and various behavioral and dietary surveys are also obtained. During a Roux-en-Y gastric bypass, a liver biopsy is routinely performed as a standard of care. From this already harvested biopsy, approximately one-third (0.5 mm x 0.5 mm x 10 mm) is separated immediately and stored in RNA later for research use. At 6 months post operatively; liver function, any disease specific laboratory tests (*i.e*. blood sugar, lipid profile, iron studies, thyroid function studies), as well as parathyroid hormone levels for adequate calcium intake are obtained.

Recruitment rates have averaged about 90% since IRB approval was obtained last fall. Currently, approximately 10 patients undergo bariatric surgery per week. Consent has been obtained from over 450 patients in the first year of the study and over 225 liver samples have been banked (discrepancy due to the 6- 8 month pre-surgical program patients must complete prior to surgery). In addition to the prospective recruitment of patients enrolling in the bariatric surgery program, the more than 800 patients who have already undergone bariatric surgery and on whom liver histology is known, are also being recruited to obtain DNA and serum samples.

### 2. Liver biopsies.

Liver biopsies are obtained by the surgeons at the same anatomic location, approximately 10 cm lateral and to the left of the falciform ligament. The tissue is then sectioned so that one-fourth to one-third is submerged directly in RNAlater (Ambion, Austin TX) in a pre-labeled plastic centrifuge tube within 3 minutes of intra-operative biopsy. The liver biopsy samples are transported from the operating room to the Geisinger Clinic Genomics Core laboratory located in the Weis Center, just across the street from the hospital. A portion of the preserved liver biopsy is removed for RNA isolation, the remainder stored in a bar-code labeled tube at -80°C that is tracked with repository management software.

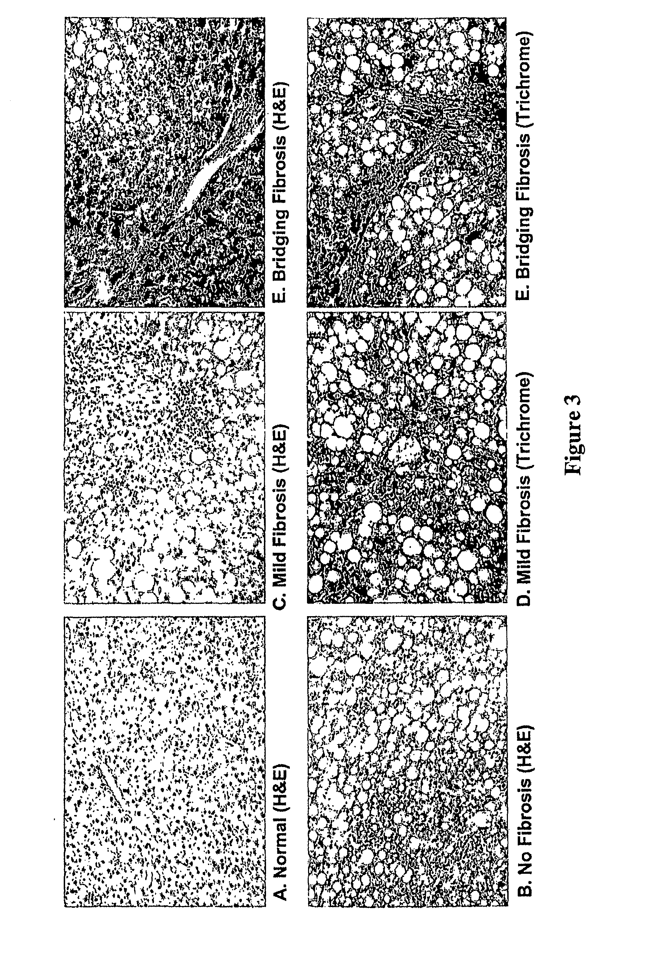

Portions of the liver biopsies are used to assess liver histology. One pathologist reads all of the liver biopsy slides from the bariatric surgery program patients, who uses the criteria proposed by Brunt, E.M., et al. (1999) Am. J. Gastroenterol. 94(9):2467-2474). Masson's trichrome is employed to detect the initial stages of perisinusoidal fibrosis that may not be discernible on routine H and E staining. Based on data from the first 200 patients, the spectrum of histological findings in NASH (representative examples in Fig. 3 with blue staining fibrosis in D and F) is:
Normal histology: 22%
Steatosis ONLY (micro or macro, any severity): 31%
Steatohepatitis (any severity)
   NO Fibrosis: 22%
   Fibrosis (portal or perivenular): 20%
Bridging Fibrosis: 5%

### 3. Clinical database

All of the extensive clinical data, such as demographics, physical findings, co-morbidities, medical history, laboratory measures, behavioral and social surveys, and drug history are entered in a research database in SAS. A majority of the patients are female with a median age of 46 and an average BMI of about 55. Specimens collected include additional blood samples for DNA isolation, serum samples, and liver tissue obtained from the intra-operative biopsy. The clinical program has been optimized, with much attention paid to maintaining the highest quality of care, to obtain clinical data with research grade quality requirements in mind. Much of this data is extracted from the electronic health record using tools that have been built by the Geisinger Clinic Information Technology personnel. Data collection is also prospective enabling longitudinal studies, important for future biomarker validation studies. For example, a NASH biomarker measured in the blood may be followed during post-surgical weight loss. This rich database also provides an opportunity to analyze gene expression data in light of a wide variety of secondary variables.

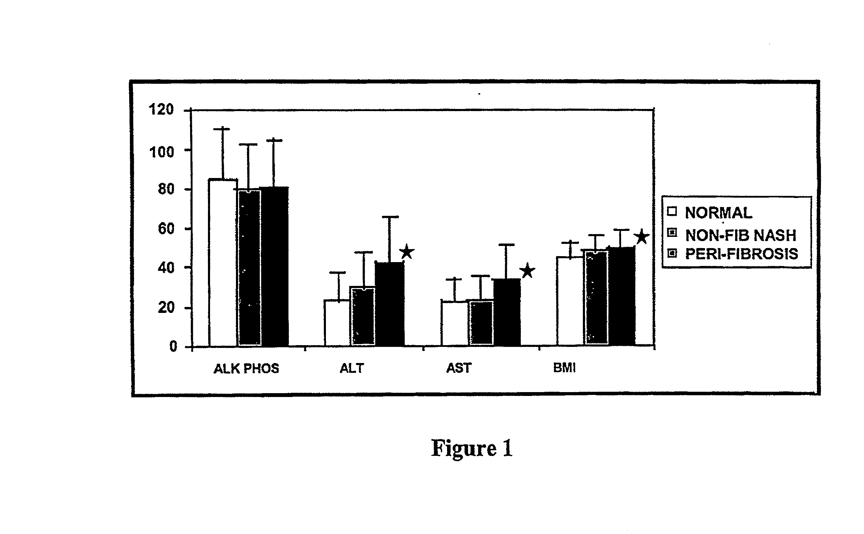

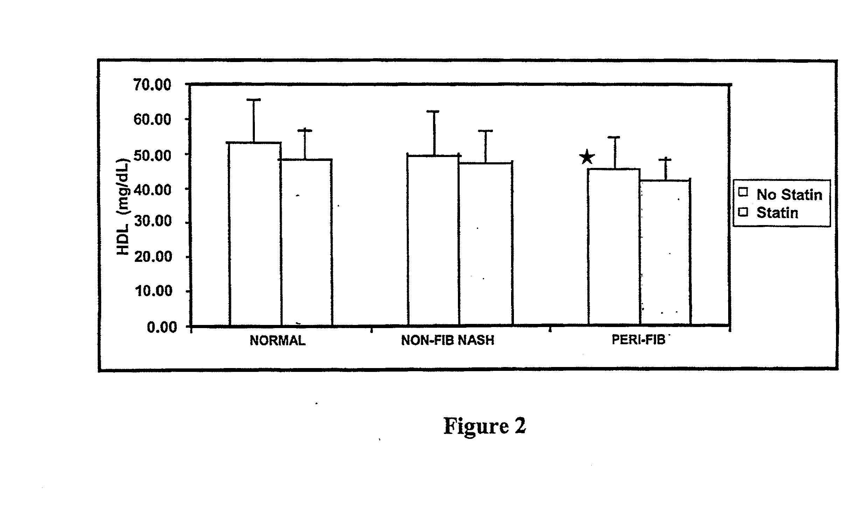

Pathology findings have been converted into a database accessible format, using a simple scoring system for levels of steatosis, lobular inflammation, fibrosis that enables statistical analysis. Initial analyses on the first extraction of the electronic medical record (n=164) have focused upon a variety of clinical laboratory parameters in patients with normal liver histology (n=44), NASH without perisinusoidal fibrosis (n=85), and perisinusoidal fibrosis (n=35). As expected, the liver function tests AST (aspartate amino transferase) and ALT (alanine amino transferase) were significantly elevated with perisinusoidal fibrosis, consistent with the expected more severe liver injury, while alkaline phosphatase was not different among groups (Fig. 1). BMI was also correlated with perisinusoidal fibrosis, as was HDL, insulin, hemoglobin A1C, glucose, and triglyceride (data not shown). Age, total cholesterol, LDL, ferritin, calcium, parathyroid hormone, albumin, folic acid, iron binding capacity, potassium, sodium, zinc, TSH, bun, creatinine, iron, and transferring saturation were not related to perisinusoidal fibrosis. The effect of statin drug use on the relationship of HDL cholesterol to perisinusoidal fibrosis was examined (Fig. 2). Statin therapy included atorvastatin, crestor, fluvastatin, lescol, lipitor, lovastatin, mevacor, pravachol, pravastatin, rosuvastatin, simvastatin, or zocor. Interestingly, in patients who were not on statins, HDL levels were decreased with perisinusoidal fibrosis, while no difference was present with statin use. Serum cholesterol (total) and LDL levels did not vary among the 3 groups (data not shown). Analysis of other clinical parameters, such as comorbidities and behavioral survey data, is ongoing.

### 4. NASH gene expression profiling

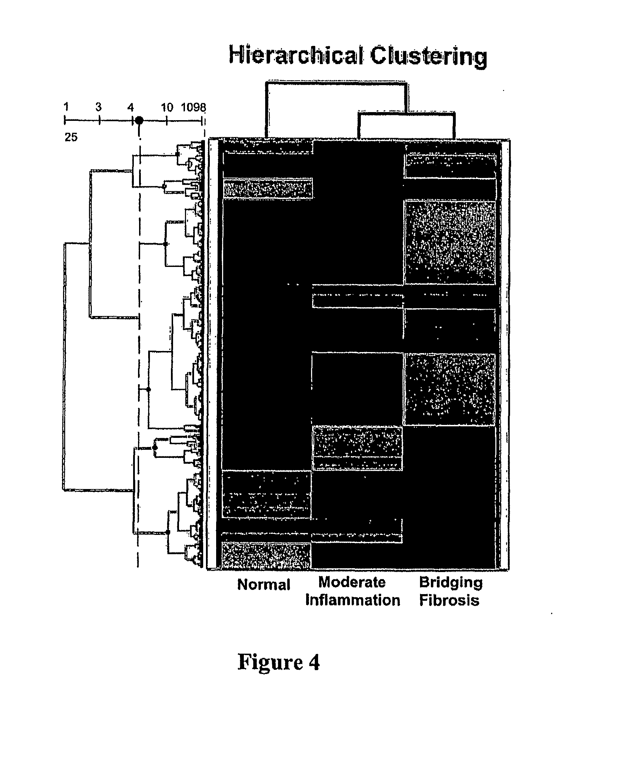

Gene expression profiles have been obtained on several individual RNA samples from patients with different subtypes of NASH (data not shown), as well as on pools of samples from normal, steatohepatitis, and bridging fibrosis (Fig. 3). using Affymetrix microarrays. Total RNA was prepared, labeled and hybridized to GeneChip Human Genome U133 Plus 2.0 Arrays as described in aim 1 below. Samples were selected from the first 190 patients recruited. Following washing and scanning, the data was analyzed using Spotfire gene expression analysis software. Both individual and pooled samples indicate that bridging fibrosis is characterized by a distinct gene expression profile relative to normal liver histology (Fig. 4).

Analysis of pools from bridging fibrosis (n=9, selected for similar histology) indicated that about 100 genes were up-regulated by at least 4-fold (signal log ratio of at least 2) relative to normal histology (n=9). Rank ordering these genes by expression level (cut-off of 100 arbitrary fluorescent units) resulted in about 40 genes (Table I). Included in this group are genes expected to be up-regulated with fibrosis including collagens type I alpha 2 and type XV alpha 1, matrix Gla protein, and EGF-containing fibulin-like extracellular matrix protein 1. In addition, interleukin 8 was upregulated, previously reported to be increased in the blood of NASH patients (Bahcecioglu, I.H. et al. (2005) Hepatogastroenterology 52(65):1549-1553). The expression levels of several small inducible cytokines were also increased. These are high priority candidate biomarkers due to their expression level, potential detection in blood, and available reagents for measurement.

### 5. Candidate gene

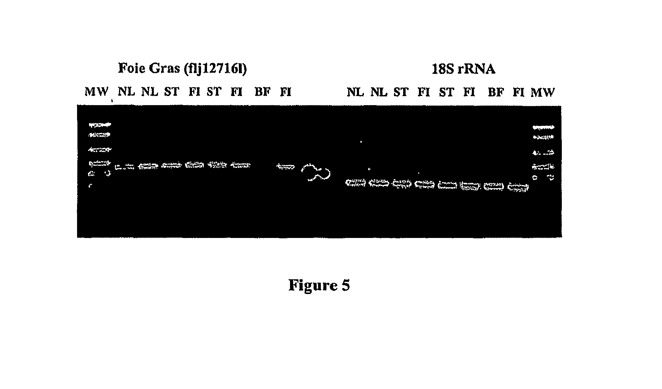

A null mutation of the *foie gras* gene in zebrafish resulted in fat accumulation in the liver of embryonic fish (Sadler, K.C. et al. (2005) Development 132(15):3561-3572). We examined our microarray gene expression data and found that 4 probes for the *foie gras* (or *flj1*2*716l*) gene were resident on the Affymetrix GeneChips. The most highly expressed of the 4 probes was found to be down-regulated by about 2 fold in a patient with bridging fibrosis. We designed PCR primers to detect this isoform and analyzed hepatic RNA from patients with normal histology, steatosis, non-bridging fibrosis, and bridging fibrosis (Fig. 5). The patient with bridging fibrosis was decreased to a level consistent with the microarray results. While only a limited sample size, and the RTPCR run under semi-quantitative conditions, we conclude that *foie gras* gene expression does not appear to be significantly altered in other histological subtypes of NASH.

**TABLE 1. Genes whose expression was up-regulated by bridging fibrosis (BF) versus normal histology (NL).**

| **NL SIGNAL** | **BF SIGNAL** | **LOG2 BF/NL** | **GENE** |
|---|---|---|---|
| 2645 | 11098 | 2.2 | *Homo sapiens* immunoglobulin A1-A2 lambda hybrid GAU heavy chain |
| 379 | 1497 | 2 | *Homo sapiens* natural killer cell transcript 4 (NK4) |
| 235 | 1393 | 2.4 | Human Ig rearranged kappa-chain gene V-J-region |
| 232 | 1301 | 3 | *Homo sapiens* diubiquitin (UBD) |
| 313 | 1242 | 2 | *Homo sapiens* GTP-binding protein overexpressed in skeletal muscle (GEM) |
| 198 | 1194 | 2.2 | *Homo sapiens* interleukin 8 (IL8) |
| 117 | 1166 | 3.4 | *Homo sapiens* defensin, alpha 1, myeloid-related sequence (DEFA1) |
| 260 | 1030 | 2.2 | *Homo sapiens* prostaglandin D2 synthase (21kD, brain) (PTGDS) |
| 167 | 948 | 2.5 | Human small inducible cytokine subfamily A (Cys-Cys), member 19 (**CCL19**) |
| 9 | 896 | 5.9 | *Homo sapiens* small inducible cytokine subfamily A (Cys-Cys), member 20 (SCYA20) **(CCL20)** |
| 275 | 851 | 2.2 | Human nephropontin (secreted phosphoprotein 1, osteopontin, bone sialoprotein I, early T-lymphocyte activation 1) |
| 197 | 831 | 2.1 | *Homo sapiens* fibulin 5 (FBLNS) |
| 66 | 778 | 3.8 | *Homo sapiens* aldo-keto reductase family 1, member B10 (aldosereductase) (AKR1B10) |
| 179 | 768 | 2.1 | *Homo sapiens* matrix Gla protein (MGP) |
| 251 | 727 | 2 | Human active IgK chain from GM 607, V-kappa-2 region |
| 119 | 589 | 2.3 | Olfactory receptor, family 2, subfamily I, member 6 |
| 129 | 497 | 2 | *Homo sapiens* collagen, type I, alpha 2 (COLIA2) |
| 80 | 430 | 2.6 | *Homo sapiens* DNA sequence from PAC 845024 on chromosome 1p36.1-36.2. |
| 124 | 414 | 2.2 | Interleukin 7 receptor |
| 110 | 407 | 2.1 | *Homo sapiens* cDNA, 3 end /clone=IMAGE-301723 |
| 48 | 347 | 2.3 | *Homo sapiens* interleukin 8 C-terminal variant (IL8) |
| 84 | 342 | 2.2 | *Homo sapiens* small inducible cytokine subfamily A (Cys-Cys), member 21 (SCYA21) **(CCL21)** |
| 76 | 333 | 2.1 | *Homo sapiens* small inducible cytokine subfamily B (Cys-X-Cys), member 6 (granulocyte chemotactic protein 2) (SCYB6) **(CXCL6)** |
| 34 | 224 | 2.3 | *Homo sapiens* clone KM36 immunoglobulin light chain variable region |
| 7 | 223 | 4.3 | *Homo sapiens* superiorcervical ganglia, neural specific 10 (SCGN10) |
| 66 | 208 | 2.1 | Human heat shock protein 70kD protein 2 |
| 43 | 203 | 2.1 | *Homo sapiens* EGF-containing fibulin-like extracellular matrix protein 1 (EFEMP1) |
| 5 | 193 | 5.3 | Superiorcervical ganglia, neural specific 10 |
| 18 | 192 | 3 | Hemoglobin, alpha 2 |
| 9 | 188 | 3.9 | *Homo sapiens* immunoglobulin lambda variable 3-10 |
| 52 | 186 | 2.4 | *Homo sapiens* pyruvate kinase, muscle (PKM2) |
| 9 | 167 | 4 | *Homo sapiens* reticulon 1 (RTN1) |
| 10 | 144 | 3.4 | *Homo sapiens* lymphocyte-specific protein tyrosine kinase (LCK) |
| 5 | 113 | 4.1 | Human Ig rearranged mu-chain gene V-N-D-N-J-region |
| 20 | 110 | 2.2 | *Homo sapiens* normal mucosa of esophagus specific 1 (NMES1) |
| 11 | 101 | 3.2 | *Homo sapiens* collagen, type XV, alpha 1 ' (COL15A1) |

### 6. Research Design and Methods

### Overview and rationale

The study is modeled on the processes used for biomarker development (Sullivan Pepe, M. et al. (2001) J. Natl. Cancer Inst. 93(14):1054-1061) in the Early Detection Research Network (EDRN) of the National Cancer Institute (Srivastava, S. and B.S. Kramer (2000) Lab. Invest. 80(8):1147-1148). Most cancers and NASH are defined histologically. Thus, tissue morphology plays a central role in the design of the study. Similar applications of gene expression profiling in cancer biology have resulted in diagnostic and prognostic reclassification and identification of new therapeutic targets (Wadlow, R. and S. Ramaswamy, (2005) Curr. Mol. Med. 5(1):111-120). In contrast to the potentially difficult goal of detecting cancers at an early stage, when the mass of tissue may be very small, the liver is a large organ that is largely, if not uniformly, affected by NASH.

Replication of the genes identified by pooled microarray analyses is validated in individual RNA samples by RT-PCR and developed as protein biomarkers for testing in serum.

The study is focused on the gene expression profile that defines a unique molecular signature for NASH related fibrosis. Published criteria for evaluating the histologic components of NASH that reflect fibrosis are shown in Table 2 (Brunt, E.M. et al. (1999) Am. J. Gastroenterol. 94(9):2467-2474). While concordance of pathologists in assessing the individual features of NASH proves challenging, this study implemented a system similar to that used previously for mouse studies (Sinclair, P.R. et al. (2003) Hepatology 37(2):351-358), for blinded readings by two pathologists. Currently, one pathologist reads all of the liver biopsies from bariatric surgery patients as part of routine standard of care. The Principal Investigator then reviews the slides following the primary diagnostic reading in a blinded fashion, and comparing findings with the pathology report on each patient. Any significant discrepancies is then be resolved by conferring with the primary pathologist and gathering opinions from other staff pathologists as required.

### A. Patient groups.

Gene expression profiling is performed on pooled RNA samples from patients with bridging fibrosis as well as severe fibrosis (perisinusoidal and/or portal). Importantly, a large number of samples with normal histology from obese patients are available. Patients will be matched for age, gender, and major clinical features, such as co-morbid conditions, to the extent possible. We expect that pools will consist of at least 10 patients for bridging fibrosis, and >25 for severe fibrosis.

### B. Microarray methods.

Total RNA is isolated from liver biopsy tissue using the RNeasy total RNA isolation kit (Qiagen). After RNA sample labeling, gene expression is examined by using the GeneChip human genome U133 plus 2.0 array (Affymetrix, Santa Clara, CA), that covers >47,000 transcripts and variants from the human genome. Due to built-in redundancy in the array design, multiple different probe sets sometimes map to the same gene or transcript, contributing to a final total of 39,000 best-characterized human genes on the HG-U133 array. All reagents used to make the final hybridization cocktail are from the Affymetrix GeneChip Expression 3' Amplification One-Cycle Target Labeling and Control Reagents Kit (Affymetrix, Santa Clara, CA). RNA targets (biotin-labeled RNA fragments) will be produced from 1-10 µg of total RNA by double-stranded cDNA synthesis followed by an *in vitro* transcription reaction and a fragmentation reaction. Hybridization cocktails containing fragmented cRNA, array controls (Affymetrix, Santa Clara, CA), BSA, and herring sperm DNA is prepared and hybridized to the array at 45°C for 16 hours. The hybridized arrays are washed, and bound biotin-labeled cRNA detected with a streptavidin-phycoerythrin conjugate. Washing and staining procedures are automated using the Affymetrix Fluidics Station (model 450). Each array will be scanned once by using the Affymetrix Gene Array Scanner 3000. The Affymetrix GeneChip Operating Software Ver 1.2 adjusts for artifacts, noise, and background. The global method of scaling/normalization will be used to for each chip. Fluorescent signal values from the Affymetrix platform are exported to the Spotfire software application (on the world-wide web with a URL address of "spotfire.com") for analysis. Normalization for chip-to-chip variation, probe-to-probe variability with Z-score and ANOVA is performed to identify genes with significantly changed expression.

**Table 2. Grading and Staging of NASH**

| **GRADING** |
|---|
| **Grade 1, mild Steatosis:** predominantly macrovesicular, ranges from less than 33% to up to 66% of the lobules Ballooning: occasionally observed; zone 3 hepatocytes Lobular inflammation: scattered and mild acute (polymorphs) and chronic inflammation (mononuclear cells) Portal inflammation: none or mild |
| **Grade 2, moderate Steatosis:** any degree, usually mixed macrovesicular and microvesicular Ballooning: present in zone 3 Lobular inflammation: polymorphs may be noted associated with ballooned hepatocytes, and/or pericellular fibrosis; mild chronic inflammation Portal inflammation: none, mild to moderate |
| **Grade 3, severe (florid steatohepatitis) Steatosis:** usually 66% (zone 3 or panacinar); commonly mixed steatosis Ballooning: predominantly zone 3; marked Lobular inflammation: scattered acute and chronic inflammation; polymorphs may appear concentrated in zone 3 areas of ballooning and perisinusoidal fibrosis Portal inflammation: mild or moderate; not predominant or marked |

| **STAGING** |
|---|
| **Staging (requires Masson trichrome or equivalent stain):** a separate portal based process with sparing of zone 3 has been proposed but remains to be established or refuted |
| **Stage 1:** Zone 3 perivenular, perisinusoidal, or pericellular fibrosis; focal or extensive |
| **Stage 2:** As for stage 1 plus focal or extensive portal fibrosis |
| **Stage 3:** Bridging fibrosis, focal or extensive |
| **Stage 4:** Cirrhosis with or without residual perisinusoidal fibrosis |

### C. Pooling.

A pooled design with a minimum of 10 samples per pool and two arrays per pool (at least 20 samples for each group) is used for the study. One concern regarding pooling is that due to the loss of degrees of freedom, a large increase in the number of samples is required to achieve a power comparable to that of a non-pooled design (Shih, J.H., et al. (2004) Bioinformatics 20(18):3318-3325). Pooled designs have also been criticized because of the inability to appropriately transform data, remove outliers, and estimate population variation. These concerns are relevant when sample size is limiting or obtaining samples is costly. Thus, any savings on microarrays may be negated. With the availability of large numbers of samples, the potential for inappropriate representation is avoided (Glass, A. et al. (2005) Biosci. Biotechnol. Biochem. 69(6):1098-1103). Pooling has been shown to reduce overall variability and result in true biological averaging for most genes (Kendziorski, C. et al. (2005) Proc. Natl. Acad. Sci. USA, 2005. 102(12):4252-4257). Thus, potential nonlinearities introduced in the generation and processing of microarray data that may cause discrepancies between average RNA abundance in a pool and the average of the same individually analyzed RNA samples appears not to be an important factor. For small designs in which only a few arrays are used in each biological condition, pooling dramatically improves accuracy, which can further be improved with biological replicates. This is consistent with our experience with the Affymetrix platform; hybridization, washing, staining, and scanning does not cause a significant amount of technical variation. We have also found that the variability among RNA samples isolated using the Qiagen kits is also minimal, *i.e.*, the RNA is usually of high quality. Biological replicates include independent pools of the same histology. Biological replication may also occur with the analysis of severe fibrosis and bridging fibrosis, which may share similar changes in gene expression.

### D. Microarray data analysis.

In addition to analysis by Spotfire, a one-gene-at-time differential analysis is performed according to method of Significance Analysis of Microarrays (Tusher, V.G. et al. (2001) Proc. Natl. Acad. Sci. USA 98(9):5116-5121) with the genes ranked by q value and with False Discovery Rate controlled for at 10% (Benjamini, Y. et al. (2001) Behav. Brain Res. 125(1-2):279-284). This software is an add-on package for the statistical program R.

### E. Potential problems and alternative approaches.

The logistical problems regarding tissue collection, RNA preservation, and microarray performance have been worked out in previous studies. Because the sizes of the biopsies obtained intra-operatively are much larger than the needle biopsies commonly performed in the outpatient setting, abundant RNA and tissue is available for analysis. Importantly, the corresponding histology is reviewed to ensure that the tissue is intact. Due to substantial cautery artifact in initial samples, surgeons now use only inscisory methods for liver biopsy.

To confirm changes in gene expression detected by pooled microarray analysis, gene expression changes of selected genes are measured using real time PCR (RT-PCR). A simple quantitative Reverse-Transcriptase Polymerase Chain Reaction (RT-PCR) using SYBR Green I, a DNA double-strand specific dye whose fluorescence is greatly enhanced by its binding to dsDNA, is used. SYBR Green I binds to the amplified PCR products during each cycle and can easily be detected by its fluorescence. Sequences for primers and probes are selected using the program Primer Express (ABI). PCR reactions for target gene and endogenous control amplicons are performed as described previously (Malek, R.L. et al. (2004) Comp. Biochem. Physiol. C. Toxicol. Pharmaco/. 138(3):363-373). For all PCR experiments, an aliquot of pooled human liver cDNA is used to provide a consistent standard across experiments. PCR expression data are analyzed using standard unpaired t-tests, with p<0.05 deemed significant. Secondary analysis includes regression analysis (now that the dimensionality will have been vastly reduced) to determine the contribution of age, sex, BMI, and other laboratory and clinical parameters to the expression of individual genes.

Statistical, clinical, and biological variables are used to select genes whose corresponding proteins have a high likelihood of detection in serum using high-throughput methods.

Candidate biomarkers are evaluated, selected, and prioritized from the genes found to be dysregulated. The liver is a major source of proteins that are present in blood, thus the protein products derived from NASH dysregulated genes may also be detectable as normal constituents in serum or plasma. In addition, ballooning degeneration of hepatocytes are a hallmark of NASH, and cell death with release of proteins into the blood may be another mechanism by which protein biomarkers derived from liver genes may be found in the circulation. The inflammatory component of NASH may also be associated with a number of soluble mediators, as suggested by our preliminary data. Degree of over- or under-expression, known information about the gene, and availability of reagents (*e.g*., antibodies) for marker assay development is the major factors that guides selection and prioritization of lead biomarker candidates. Based upon our data, the group of small inducible cytokines up-regulated in bridging fibrosis are the strong lead candidates.

### B. High-throughput protein-based assays.

To evaluate lead biomarker candidates, assays that can be performed in a relatively high throughput format is implemented and/or developed. Initial evaluation is performed using an immunological assay, either immunoblot or enzyme-linked immunosorbent assay (ELISA). The key reagent for either technique is an antibody corresponding to the biomarker. For genes for which no such reagent is available, antisera will be generated against a peptide synthesized from a region that is likely to be antigenic and available for antigen-antibody interaction. Alternatively, antibodies may be generated by immunization of animals with peptides or whole proteins using standard methods known in the art. Bioinformatic approaches are used to help identify sequences for antibody development. With an available antibody, the first phase of development is to perform an immunoblot with serum, liver, and peripheral blood immune cells. This provides basic information regarding the specificity of the antisera, as well as information about the biomarker protein in the various tissue compartments. A key aspect for quantification is an internal control (*e.g*., normal serum or plasma) that is run on each blot and prepared in from large volume and aliquoted so that it is reproducible.

The second phase is the development of an ELISA based assay. This is performed on lead biomarkers that pass the initial immunoblot analysis, or on leads for which an ELISA is already available. This highly sensitive format will also be ideal for biomarker candidates that may be present at low levels and be marginally or not detectable by immunoblot. For ELISAs, several strategies exist for devising the assay, including direct and indirect methods. The chosen strategy depends upon the availability and performance of the available antibody and antigen reagents.

### C. Biomarker assay evaluation.

The performance of the protein based assays developed above for lead biomarker candidates is evaluated on a small group of patients on whom liver histology is known. If sensitivity of detection is found to be satisfactory, *i.e*., protein can be detected in patients with NASH/fibrosis, Phase II studies will focus on validation of the marker in larger patient cohorts as well as in longitudinal studies. Since biomarker discovery is performed from cases drawn from the same population as subsequent initial evaluation, sampling bias from artifactual case control differences will be avoided. The laboratory procedures of sample collection, processing, and storage will thus be identical for future validation studies and the initial assay evaluation. Blood samples are collected under standardized conditions (*e.g*., patient fasting and in supine position in the morning).

For protein-based markers, multiple isoforms may be encountered. Thus, qualitative evaluation by immunoblot is performed to determine not only the effectiveness of the antibody as a potential reagent but also to gather some initial biological information on the biomarker protein. Potential sources of biomarker variability may be due to diurnal variation in secretion, inherent assay variability, and gender or age variation. Importantly, a biomarker may vary with different co-morbidities or medications. Thus, results from biomarker evaluation studies needs to be analyzed in the context of the clinical variables in the clinical database.

The biomarkers identified in this study are representative of morbidly patients in our study population. Extending evaluation of the biomarkers to other populations, with various levels of obesity, will be important for continued validation. The sensitivity and specificity of a molecular marker cannot be fully realized until careful testing is carried out in large numbers of specimens and compared with controls. Validation studies in large cohorts of patients, including patients at high risk for developing NASH fibrosis, is an important step toward clinical use. A critical component of these validation studies is the assessment of outcome, *i.e*., whether tests using these new molecular markers detect NASH fibrosis at an early enough stage to prevent the serious sequelae, including cirrhosis and hepatocellular carcinoma.

### Example 2

Changes in expression of two lead candidate biomarkers from preliminary microarray expression data have been confirmed by RT-PCR. Based upon related data from the literature (Asselah, T. et al. (2005) Gastroenterology 129(6):2064-2075; Bonacchi, A. et al. (2003) Gastroenterology 125(4):1060-1076), these molecules are two lead candidates for subsequent development as diagnostic biomarkers for NASH related fibrosis.

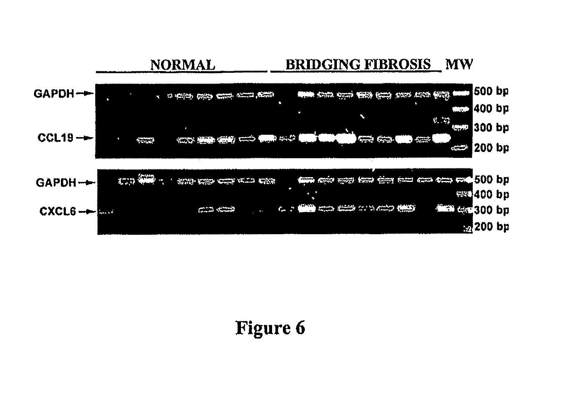

Several genes with increased expression in NASH related hepatic fibrosis were selected for further study based upon the microarray gene expression data shown in **Table 1.** The presence of 4 small inducible cytokines was of interest because of their inducibility and corresponding potential for high specificity as a diagnostic test, and their solubility and corresponding potential for detection in peripheral blood. Two of these were selected for further study based upon analysis of microarray results from patients with NASH but without fibrosis (data not shown). Because the initial microarray results were performed on pooled samples, reverse transcriptase PCR was performed on RNA from each of the individual patients in the pools to amplify small inducible cytokine subfamily A (Cys-Cys), member 19 (CCL19) and small inducible cytokine subfamily B (Cys-X-Cys), member 6 (CXCL6). As shown below in Fig. 6, each of the genes was easily detectable. GAPDH was used as a control gene, which has been used by others for analysis of gene expression in fibrotic liver samples from patients with hepatitis C (Bonacchi, A., I. et al. (2003) Gastroenterology 125(4):1060-1076), and whose expression was found to be unchanged by fibrosis in our microarray results (data not shown). These data provide the basis for implementing fluorescent quantitative real time PCR to provide a more accurate quantification of relative expression levels.

In lieu of this real time PCR data, an estimate of expression levels was obtained by digital imaging and quantification of the PCR bands. The mean CCL19/GAPDH ratio was 1.45 in patients with normal liver histology and 2.17 in patients with bridging fibrosis. The mean CXCL6/GAPDH ratios in normal and bridging fibrosis were 0.542 and 1.35, respectively. These values likely underestimate the actual ratios, especially for the CCL19, where several of the bands are strong and are beyond the linear range of the PCR.

The CCL19/GAPDH and CXCL6/GAPDH ratios for each patient were then correlated with several pathological and serological parameters (Table 3). The two genes had a 0.53 correlation (r) with each other. Interestingly, neither showed a significant correlation with the degree of inflammation present in the liver and were widely discordant for degree of steatosis and portal fibrosis. The strongest correlations were between CCL19 and LDL (-0.88), CCL19 and ferritin (-0.69), and CXCL6 and insulin (+0.61). These data indicate that the two cytokines may have very different roles in the pathophysiology of NASH related fibrosis. The analyses also provide potential avenues to pursue for future studies.

**TABLE 3. Correlations between gene expression and serum or NASH pathology parameters.**

| **GENE** | **ST** | **INFL** | **VF** | **PF** | **FERR** | **HDL** | **LDL** | **TRI** | **INS** | **GLU** | **A1C** |
|---|---|---|---|---|---|---|---|---|---|---|---|
| **CCL19** | -0.49 | -0.05 | -0.15 | -0.12 | -0.69 | -0.44 | -0.88 | -0.21 | 0.27 | 0.44 | 0.50 |
| **CXCL6** | 0.25 | -0.00 | -0.39 | 0.34 | -0.03 | -0.57 | -0.45 | -0.58 | 0.61 | -0.37 | -0.28 |

| | | | | | | | | | | | |
|---|---|---|---|---|---|---|---|---|---|---|---|
| ST= steatosis grade; INFL=inflammation grade; VF=peri-venular fibrosis grade; PF=portal fibrosis grade; FERR=serum ferritin; HDL=high density lipoprotein; LDL=low density lipoprotein; TRI=triglyceride; INS=insulin; GLU=glucose; AlC=hemoglobin AlC. | | | | | | | | | | | |

Interestingly, both CCL19 and CXCL6 have been previously implicated in hepatic fibrosis due to hepatitis C infection. The expression of the CXCL6 was found to be up-regulated in RNA from mildly to severely fibrotic liver samples from patients with chronic hepatitis C infection (Asselah, T. et al. (2005) Gastroenterology 129(6):2064-2075). The expression of the receptor for CCL19, CCR7, as well as the small inducible cytokine CCL21, has been proposed to be involved in the promotion of fibrogenesis from activated hepatic stellate cells in hepatitis C infection (Bonacchi, A., et al. (2003) Gastroenterology 125(4):1060-1076). The finding of dysregulation of these cytokines in both NASH fibrosis and in hepatitis C fibrosis adds support to their potential involvement in the process of fibrosis, and to their potential utility as biomarkers of fibrosis.

### Example 3

### Detection of NASH markers in peripheral blood RNA

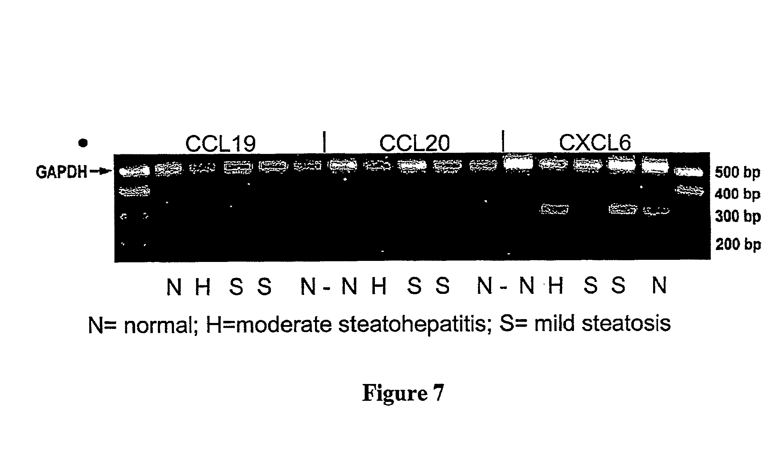

Expression of three genes found to be over-expressed in liver samples with NASH related bridging fibrosis was analyzed by reverse transcriptase PCR in RNA derived from peripheral blood mononuclear cells obtained from morbidly obese patients with NASH. The small inducible cytokine subfamily A (Cys-Cys), member 19 (CCL19), member 20 (CCL20) and small inducible cytokine subfamily B (Cys-X-Cys), member 6 (CXCL6) were amplified and are shown below in Fig. 7, each of the genes was easily detectable. GAPDH was used as a control gene. Bands were easily detected for two of the cytokines, but not for CCL20. These results indicate that genes whose expression is found to be altered in liver RNA can also be measured in RNA obtained from circulating peripheral blood mononuclear cells.

### Plasma and Serum Collection.

Whole blood was collected from morbidly obese patients by venipuncture into Cell Processing Tubes (CPT, Becton Dickinson) or into tubes without anticoagulant (Vacutainer tubes; Becton Dickinson, Franklin Lakes, N.J.). No screening of donors was attempted with respect to age, diet or other variables. All volunteers were informed of the study and gave their written consent. The blood was centrifuged at 1160xg for 10 min. The plasma and serum were aliquoted and stored frozen at -80°C until assay.

### Example 4

### Detection of NASH markers in serum.

The CCL20 ELISA kit (R and D Systems, Minneapolis, MN) was used to measure CCL20 protein levels in the serum of morbidly obese patients with normal liver histology (n=3), moderate steatohepatitis (n=4) and bridging fibrosis (n=8). For this assay, 100 µl of Assay Diluent RD1-57 was added to each well of a microplate strip. 100 µl of Standard, sample, or control was added to each well. The wells were covered with the adhesive strip provided and incubated for 2 hours at room temperature. Each well was then aspirated and washed, repeating the process three times for a total of four washes. Washes were done by filling each well with Wash Buffer (400 µl) using a squirt bottle, and after the last wash, removing any remaining Wash Buffer by aspirating. 200 µl of MIP-3 conjugate was then added to each well, covered with a new adhesive strip, and incubated for 2 hours at room temperature. The aspiration/washes were then repeated. 200 µl of Substrate Solution was added to each well, incubated for 30 minutes at room temperature, protected from light. 50 µl of Stop Solution was then added to each well. The optical density of each well was measured in a microplate reader within 30 minutes, set to 450 nm. Wavelength correction was used at 540 nm.

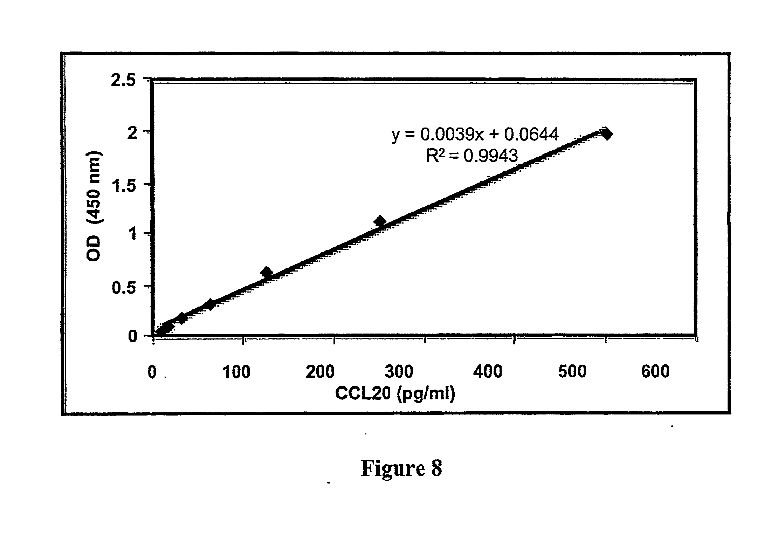

The standard curve for the CCL20 ELISA assay is shown in **Fig. 8****.** The linearity and sensitivity indicates that the assay is very robust.

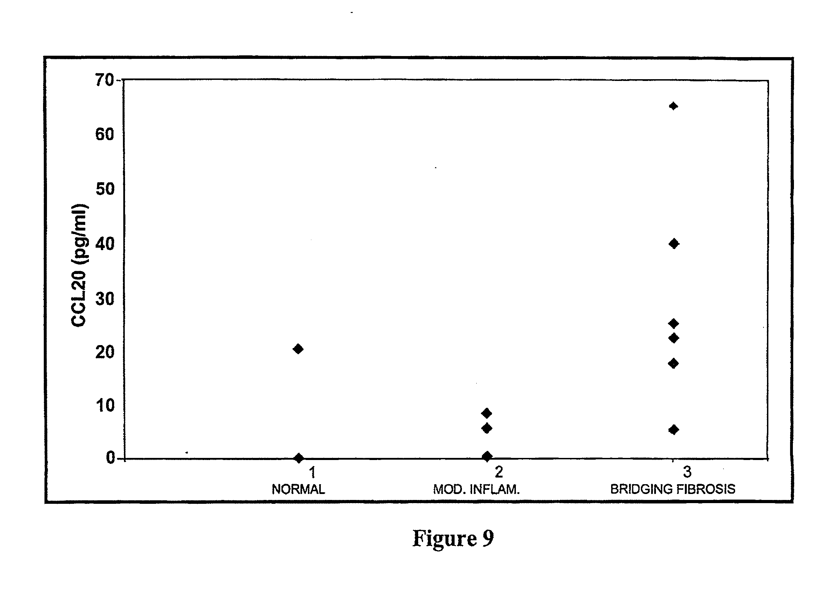

Serum samples from morbidly obese patients were then analyzed from patients with normal histology, with moderate inflammation, and with bridging fibrosis. As shown in **Fig. 9****,** patients with normal liver histology, or with moderate inflammation had lower levels of CCL20 protein in their serum than patients with bridging fibrosis. CCL20 was not detected in peripheral blood mononuclear cell RNA, thus is likely derived at least in part, or predominantly, from liver.

### Example 5

### Expression confirmation of lead candidate CCL20.

A small inducible cytokine gene (small inducible cytokine subfamily A (Cys-Cys), member 20 (CCL20)) was selected as the initial lead candidate to pursue since it was the most up-regulated gene present in the list that met the initial selection criteria and its expression was confirmed using semi-quantitative PCR. GAPDH was used as a control gene, as it is used in the art for analysis of gene expression in fibrotic liver samples from patients with hepatitis C (Bonacchi, A., et al. (2003) Gastroenterology 125(4):1060-1076), and whose expression was found to be unchanged by fibrosis in our microarray results (data not shown). Real-time PCR was performed on approximately 12 RNA samples from liver tissue with normal histology and 12 RNA samples with bridging fibrosis using a Taqman assay on an ABI 7300 real time PCR instrument. GAPDH was again used as a control. As shown in **Fig. 10A****,** CCL20 (black lines) exhibited a range of expression levels across patients with normal histology and severe (bridging) fibrosis. GAPDH levels (grey lines) were extremely consistent across such diverse samples as evident in the tight grouping of fluorescence plots, further validating its use as a control for gene expression studies in NASH. Using GAPDH mRNA levels as a reference housekeeping control gene, expression of CCL20 was much less in all but one of the normal histology samples than in the bridging fibrosis samples **(****Fig. 10B****).** This data confirms the Affymetrix chip data.

### Example 6

### Detection of CCL20 in serum.

An ELISA kit for CCL20 was purchased (R & D Systems, MN) to analyze levels in the serum of NASH patients. CCL20 protein levels were measured in 30 serum samples from patients with normal liver histology, and 35 with moderate to severe fibrosis, including 4 patients with cirrhosis. Using a threshold of 14 pg/ml, the predictive power to detect NASH fibrosis was 83% versus only 9% for LFTs (elevation of AST and/or ALT) using the clinical guideline for liver biopsy of 2 times the upper limit of normal. If, instead, a much more conservative threshold (>80 u/ml) is used, the predictive power improves to only 15%. Even if the upper limit of the reference ranges for either ALT or AST are used, which would not be used to initiate a liver biopsy, the positive predictive value is still <25%. Importantly, all 4 patients with cirrhosis in this cohort had normal LFTs but elevated CCL20 levels.

Clinical utility of CCL20 versus LFTs for mild to severe NASH fibrosis in a 65 patient cohort

| **Positive Predictive Value** | | **Negative Predictive Value** |
|---|---|---|
| **CCL20** | 81 % | 79% |
| **LFTS (>2X)** | 9% | 47% |
| **LFTs (>80)** | 15% | 49% |
| **LFTs (>NL)** | 24% | 52% |

### Example 7

### Analysis of peripheral blood mononuclear cell mRNA.

In addition to serum, the cellular component of peripheral blood is also useful for biomarker analysis. Since the majority of white blood cells circulating in the periphery are neutrophils, which have a very short life span, are very reactive to multiple stimuli, and are usually not a major component of NASH in the liver, we chose to isolate mRNA from peripheral blood mononuclear cells (PBMCs). These cells include lymphocytes and monocytes, which constitute the majority of the inflammatory component in NASH. We used semi-quantitative conventional PCR for CCL19, CCL20, and CXCL6 to determine whether the mRNA from these genes could be detected in the circulating PBMCs from NASH patients. Bands were detectable for CCL19 and CXCL6 but not for CCL20 (data not shown). Because CCL20 protein was detectable in serum, its absence from PBMCs is consistent with a primarily hepatic origin of CCL20.

### Example 8

### Measurement of biomarker candidate CCL19 in RNA from Peripheral Blood Mononuclear Cells (PBMCs).

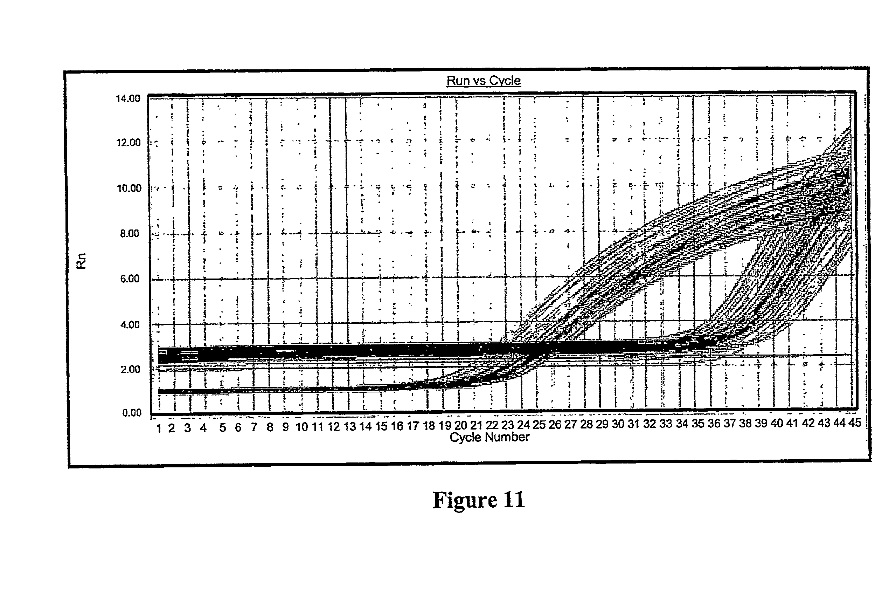

CPT tubes were used to collect PBMCs on an initial 100 patients. CCL19 RNA levels were measured in PBMC RNA samples using a Taqman assay on an ABI 7300 real time PCR instrument from 13 patients with normal liver histology (NL), 6 patients with steatohepatitis but no fibrosis (SH), 10 patients with peri-sinusoidal fibrosis (PF), 2 patients with bridging fibrosis (BF), and one with cirrhosis (CR) (black lines, **Fig. 11**). Levels of control gene GAPDH (grey lines, **Fig. 11**) were generally consistent, reflecting high quality and integrity of sample collection and preparation. CCL19/GAPDH ratios were evaluated across patient groups. Using the second highest value from patients with normal liver histology as a threshold cut-off, values above this level would have a positive predictive value of 88.9% for NASH (encompassing SH, PF, BF or CR), with a negative predictive value of 52%. In contrast, the liver function tests AST or ALT were elevated in only 12% of these patients.

In view of the above, it will be appreciated that the invention also encompasses the following items:
1. A method for diagnosing a NASH disease state in a patient comprising determining a level of expression of a panel of genes associated with the onset or progression of NASH in a patient sample; comparing the level of expression with a predetermined value for NASH-associated gene expression in said panel of genes and correlating the level of expression with a NASH disease state.
2. The method of item 1 wherein said panel of genes includes one or more small inducible cytokines.
3. The method of item 2 wherein said small inducible cytokine is at least one selected from the group consisting of SCYA19, SCYA20, SCYA21 and SCYB6.
4. The method of item 1 wherein said panel comprises at least one gene selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (21kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2 region; Olfactory receptor, family 2, subfamily I, member 6; collagen, type I, alpha 2 (COL1A2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN10); heat shock protein 70kD protein 2; EGF-containing fibulin-like extracellular matrix protein 1 (EFEMP1); Superiorcervical ganglia, neural specific 10; Hemoglobin, alpha 2; immunoglobulin lambda variable 3-10; pyruvate kinase, muscle (PKM2); reticulon 1 (RTN1); lymphocyte-specific protein tyrosine kinase (LCK); Immunoglobulin rearranged mu-chain gene V-N-D-N-J-region; normal mucosa of esophagus specific 1 (NMES1); and collagen, type XV, alpha 1 (COL15A1).
5. The method of item 1 wherein said predetermined value of NASH-associated gene expression represents an increase over normal expression of the genes by at least 4 fold.
6. The method of item 1 wherein said predetermined value of NASH-associated gene expression represents an increase over normal expression of the genes by 4 to 12 fold.
7. The method of item 1 wherein the level of gene expression is determined by immunoassay measuring the level of protein expressed from said genes.
8. The method of item 1 wherein said patient sample is blood.
9. A method for treating NASH in a mammalian subject comprising administering to a mammalian subject suspected of having or being susceptible to NASH, a therapeutically effective amount of an inhibitor of at least one NASH-related gene or protein in an amount effective to reduce, eliminate or prevent NASH.
10. The method of item 9 wherein said mammalian subject is a human.
11. The method of item 9 wherein said inhibitor inhibits at least one small inducible cytokine.
12. The method of item 11 wherein said small inducible cytokine is selected from the group consisting of SCYA19, SCYA20, SCYA21 and SCYB6.
13. The method of item 9 wherein said inhibitor inhibits expression of at least one gene selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (21kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2 region; Olfactory receptor, family 2, subfamily I, member 6; collagen, type I, alpha 2 (COL1A2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN 10); heat shock protein 70kD protein 2; EGF-containing fibulin-like extracellular matrix protein 1 (EFEMP1); Superiorcervical ganglia, neural specific 10; Hemoglobin, alpha 2; immunoglobulin lambda variable 3.-10; pyruvate kinase, muscle (PKM2); reticulon 1 (RTN1); lymphocyte-specific protein tyrosine kinase (LCK); immunoglobulin rearranged mu-chain gene V-N-D-N-J-region; normal mucosa of esophagus specific 1 (NMES1); and collagen, type XV, alpha 1 (COL15A1).
14. The method of item 9 wherein said inhibitor is at least one antibody.
15. The method of item 14 wherein said antibody is a monoclonal antibody.
16. The method of item 14 wherein said antibody or antibodies specifically bind to at least one protein selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (21kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2 region; Olfactory receptor, family 2, subfamily I, member 6; collagen, type I, alpha 2 (COLIA2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN 10); heat shock protein 70kD protein 2; EGF-containing fibulin-like extracellular matrix protein 1 (EFEMP1); Superiorcervical ganglia, neural specific 10; Hemoglobin, alpha 2; immunoglobulin lambda variable 3-10; pyruvate kinase, muscle (PKM2); reticulon 1 (RTN1); lymphocyte-specific protein tyrosine kinase (LCK); Immunoglobulin rearranged mu-chain gene V-N-D-N-J-region; normal mucosa of esophagus specific 1 (NMES1); and collagen, type XV, alpha 1 (COL15A1).
17. A method for treating NASH in a mammalian subject comprising administering to a mammalian subject suspected of having or being susceptible to NASH, a therapeutically effective amount of an inhibitor of at least one receptor for a NASH-related protein in an amount effective to reduce, eliminate or prevent NASH.
18. The method of item 17 wherein said receptor is a receptor for a small inducible cytokine.
19. The method of item 18 wherein said small inducible cytokine is at least one selected from the group consisting of CLC19, SCYA20, SCYA21, and CXCL6.
20. The method of item 17 wherein said receptor is for a protein selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (21kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2. region; Olfactory receptor, family 2, subfamily I, member 6; collagen, type I, alpha 2 (COL1A2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN10); heat shock protein 70kD protein 2; EGF-containing fibulin-like extracellular matrix protein 1 (EFEMP1); Superiorcervical ganglia, neural specific 10; Hemoglobin, alpha 2; immunoglobulin lambda variable 3-10; pyruvate kinase, muscle (PKM2); reticulon 1 (RTN1); lymphocyte-specific protein tyrosine kinase (LCK); Immunoglobulin rearranged mu-chain gene V-N-D-N-J-region; normal mucosa of esophagus specific 1 (NMES 1); and collagen, type XV, alpha 1 (COL15A1).
21. A diagnostic tool comprising a plurality of isolated polynucleotides associated with non-alcoholic steatohepatitis immobilized on a substrate, wherein said plurality of polynucleotides comprises at least two polynucleotides that specifically bind to at least two polynucleotides that encode proteins having at least 95% identity to the proteins selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (21kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2 region; Olfactory receptor, family 2, subfamily I, member 6; collagen, type I, alpha 2 (COL1A2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN10); heat shock protein 70kD protein 2; EGF-containing fibulin-like extracellular matrix protein 1 (EFEMP1); Superiorcervical ganglia, neural specific 10; Hemoglobin, alpha 2; immunoglobulin lambda variable 3-10; pyruvate kinase, muscle (PKM2); reticulon 1 (RTN1); lymphocyte-specific protein tyrosine kinase (LCK); Immunoglobulin rearranged mu-chain gene V-N-D-N-J-region; normal mucosa of esophagus specific 1 (NMES1); and collagen, type XV, alpha 1 (COL15A1).
22. A diagnostic tool comprising a plurality of isolated polynucleotides associated with non-alcoholic steatohepatitis immobilized on a substrate, wherein said plurality of polynucleotides comprises at least two polynucleotides that specifically bind to at least two polynucleotides that encode proteins having at least 95% identity to small inducible cytokines.
23. The diagnostic tool of item 22 wherein said small inducible cytokines are selected from the group consisting of CCL19, SCYA20, SCYA21 and CXCL6.
24. A method of preventing NASH or inhibiting the progression of NASH comprising administering a therapeutically effective amount of a composition that inhibits the expression of at least two NASH-related genes.
25. The method of item 24 wherein at least one of said NASH related genes is a small inducible cytokine.
26. The method of item 25 wherein said small inducible cytokine is selected from the group consisting of CCL19, SCYA20, SCYA21 and CXCL6.
27. The method of item 24 wherein said NASH related genes are selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (2 1 kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2 region; Olfactory receptor, family 2, subfamily I, member 6; collagen, type I, alpha 2 (COL1A2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN10); heat shock protein 70kD protein 2; EGF-containing fibulin-like extracellular matrix protein 1 (EFEMP 1); Superiorcervical ganglia, neural specific 10; Hemoglobin, alpha 2; immunoglobulin lambda variable 3-10; pyruvate kinase, muscle (PKM2); reticulon 1 (RTN1); lymphocyte-specific protein tyrosine kinase (LCK); Immunoglobulin rearranged mu-chain gene V-N-D-N-J-region; normal mucosa of esophagus specific 1 (NMES 1); and collagen, type XV, alpha 1 (COL15A1).
28. The method of item 24 wherein said composition comprises an inhibitor of gene expression selected from an antisense RNA, a morpholino polynucleotide, and an interfering RNA (RNAi).
29. A method of preventing NASH or inhibiting the progression of NASH comprising identifying a patient in with NASH or at risk of developing NASH and administering a therapeutically effective amount of a composition that inhibits the biological activity of at least one NASH-related protein.
30. The method of item 29 wherein said composition comprises an antibody or small molecule that specifically binds to said NASH-related protein.
31. The method of item 30 wherein said NASH-related proteins comprise at least one small inducible cytokine.
32. The method of item 31 wherein said small inducible cytokine is selected from the group consisting of CCL19, SCYA20, SCYA21 and CXCL6.
33. The method of item 31 wherein said NASH-related proteins comprise at least one protein selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (21kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2 region; Olfactory receptor, family 2, subfamily I, member 6; collagen, type I, alpha 2 (COL1A2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN10); heat shock protein 70kD protein 2; EGF-containing fibulin-like extracellular matrix protein 1 (EFEMP1); Superiorcervical ganglia, neural specific 10; Hemoglobin, alpha 2; immunoglobulin lambda variable 3-10; pyruvate kinase, muscle (PKM2); reticulon 1 (RTN1); lymphocyte-specific protein tyrosine kinase (LCK); Immunoglobulin rearranged mu-chain gene V-N-D-N-J-region; normal mucosa of esophagus specific 1 (NMES1); and collagen, type XV, alpha 1 (COL15A1).
34. The method of item 30 wherein said inhibitor is a monoclonal antibody.
35. The method of item 30 wherein said inhibitor is a polyclonal antibody.
36. A method for screening for compounds that inhibit NASH comprising contacting a test substance with at least one protein associated with NASH and determining whether said test substance binds to said protein or its receptor, wherein binding indicates that said test substance is capable of inhibiting NASH.
37. The method of item 36 wherein said protein is a small inducible cytokine or a receptor thereof.
38. The method of item 37 wherein said small inducible cytokine is selected from the group consisting of CCL19, SCYA20, SCYA21, and CXCL6 or the receptor thereof.
39. The method of item 36 wherein said protein is selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (21kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2 region; Olfactory receptor, family 2, subfamily I, member 6; collagen, type I, alpha 2 (COL1A2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN10); heat shock protein 70kD protein 2; EGF-containing fibulin-like extracellular matrix protein 1 (EFEMP1); Superiorcervical ganglia, neural specific 10; Hemoglobin, alpha 2; immunoglobulin lambda variable 3-10; pyruvate kinase, muscle (PKM2); reticulon 1 (RTN1); lymphocyte-specific protein tyrosine kinase (LCK); Immunoglobulin rearranged mu-chain gene V-N-D-N-J-region;
   normal mucosa of esophagus specific 1 (NMES 1); collagen, type XV, alpha 1 (COL15A1) and a receptor thereof.
40. The method of item 36 further comprising determining whether said test substance inhibits the biological activity of said protein or its receptor.
41. The method of item 40 wherein said step of determining whether said test substance inhibits the biological activity of said protein or its receptor comprises a cell-based assay.
42. A kit for the diagnosis of NASH in a patient comprising at least one polynucleotide probe that specifically binds to a polynucleotide encoding a NASH-related protein, and instructions for use.
43. A kit for the diagnosis of NASH in a patient comprising at least one antibody or antigen binding fragment of an antibody that specifically binds to a NASH-related polypeptide, and instructions for use.
44. The kit of item 42 or 43 wherein said NASH-related protein is selected from the group consisting of immunoglobulin A1-A2 lambda hybrid GAU heavy chain; natural killer cell transcript 4 (NK4); Immunoglobulin rearranged kappa-chain gene V-J-region; diubiquitin (UBD); GTP-binding protein overexpressed in skeletal muscle (GEM); interleukin 8 (IL8); defensin, alpha 1, myeloid-related sequence (DEFA1); prostaglandin D2 synthase (21kD, brain) (PTGDS); small inducible cytokine subfamily A (Cys-Cys), member 19; small inducible cytokine subfamily A (Cys-Cys), member 20 (SCYA20); nephropontin (secreted phosphoprotein 1, osteopontin, bone sialoprotein I, early T-lymphocyte activation 1); fibulin 5 (FBLN5); aldo-keto reductase family 1, member B10 (aldose reductase) (AKR1B10); matrix Gla protein (MGP); IgK chain from GM 607, V-kappa-2 region; Olfactory receptor, family 2, subfamily I, member 6; collagen, type I, alpha 2 (COL1A2); DNA sequence from PAC 845024 on chromosome 1p36.1-36.2; Interleukin 7 receptor; cDNA, 3 end /clone=IMAGE-301723; interleukin 8 C-terminal variant (IL8); small inducible cytokine subfamily A (Cys-Cys), member 21 (SCYA21); small inducible cytokine subfamily B (Cys-X-Cys), member 6 (granulocyte chemotactic protein 2) (SCYB6); clone KM36 immunoglobulin light chain variable region; superiorcervical ganglia, neural specific 10 (SCGN10); heat shock protein 70kD protein 2; EGF-containing fibulin-like extracellular matrix protein 1 (EFEMP1); Superiorcervical ganglia, neural specific 10; Hemoglobin, alpha 2; immunoglobulin lambda variable 3-10; pyruvate kinase, muscle (PKM2); reticulon 1 (RTN1); lymphocyte-specific protein tyrosine kinase (LCK); Immunoglobulin rearranged mu-chain gene V-N-D-N-J-region; normal mucosa of esophagus specific 1 (NMES1); collagen, type XV, alpha 1 (COL15A1) and a receptor thereof.
45. The method of item 1 wherein said panel of genes consists essentially of CCL19.
46. The method of item 1 wherein said panel of genes consists essentially of SCYA20.
47. A diagnostic tool comprising an isolated polynucleotide associated with non-alcoholic steatohepatitis immobilized on a substrate wherein said polynucleotide comprises a polynucleotide sequence that specifically binds to a polynucleotide sequence encoding CCL19.
48. A diagnostic tool comprising an isolated polynucleotide associated with non-alcoholic steatohepatitis immobilized on a substrate wherein said polynucleotide comprises a polynucleotide sequence that specifically binds to a polynucleotide sequence encoding SCYA20.

## Claims

1. A method for diagnosing a NASH disease state in a patient comprising determining a level of expression of a panel of genes associated with the onset or progression of NASH in a patient sample; comparing the level of expression with a predetermined value for NASH-associated gene expression in said panel of genes and correlating the level of expression with a NASH disease state, wherein said panel of genes comprises at least the small inducible cytokine subfamily A (Cys-Cys), member 20 (SCYA20) (CCL20).

2. The method of claim 1, wherein
a) said predetermined value of NASH-associated gene expression represents an increase over normal expression of the genes by at least 4 fold, or by 4 to 12 fold, or
b) the level of gene expression is determined by immunoassay measuring the level of protein expressed from said genes, or
c) said patient sample is blood, or
d) said panel of genes consists essentially of SCYA20.

3. An inhibitor of at least one NASH-related gene or protein for use for treating NASH, wherein said inhibitor inhibits at least the small inducible cytokine subfamily A (Cys-Cys), member 20 (SCYA20) (CCL20).

4. The inhibitor of claim 3, wherein said mammalian subject is a human, and/or wherein said inhibitor is at least one antibody, preferably a monoclonal antibody.

5. The inhibitor of claim 4, wherein said antibody or antibodies specifically bind to at least the small inducible cytokine subfamily A (Cys-Cys), member 20 (SCYA20) (CCL20).

6. An inhibitor of at least one receptor for a NASH-related protein for use for treating NASH, wherein said receptor is a receptor for the small inducible cytokine subfamily A (Cys-Cys), member 20 (SCYA20) (CCL20).

7. A diagnostic tool comprising
a) a plurality of isolated polynucleotides associated with nonalcoholic steatohepatitis immobilized on a substrate, wherein said plurality of polynucleotides comprises at least two polynucleotides that specifically bind to at least two polynucleotides that encode proteins having at least 95% identity to small inducible cytokine subfamily A (Cys-Cys), member 20 (SCYA20) (CCL20), or
b) an isolated polynucleotide associated with non-alcoholic steatohepatitis immobilized on a substrate, wherein said polynucleotide comprises a polynucleotide sequence that specifically binds to a polynucleotide sequence encoding SCYA20.

8. A composition that inhibits the expression of at least two NASH-related genes for use for preventing NASH or inhibiting the progression of NASH, wherein at least one of said NASH related genes is the small inducible cytokine subfamily A (Cys-Cys), member 20 (SCYA20) (CCL20).

9. The composition of claim 8, wherein said composition comprises an inhibitor of gene expression selected from an antisense RNA, a morpholino polynucleotide, and an interfering RNA (RNAi).

10. A composition that inhibits the biological activity of at least one NASH-related protein for use for preventing NASH or inhibiting the progression of NASH, wherein said NASH-related proteins comprise at least the small inducible cytokine subfamily A (Cys-Cys), member 20 (SCYA20) (CCL20).

11. The composition of claim 10, wherein said composition comprises an antibody or small molecule that specifically binds to said small inducible cytokine subfamily A (Cys-Cys), member 20 (SCYA20) (CCL20), preferably, wherein said antibody is a monoclonal or a polyclonal antibody.

12. A method for screening for compounds that inhibit NASH comprising contacting a test substance with at least one protein associated with NASH and determining whether said test substance binds to said protein or its receptor, wherein binding indicates that said test substance is capable of inhibiting NASH, wherein said protein is a small inducible cytokine subfamily A (Cys-Cys), member 20 (SCYA20) (CCL20) or a receptor thereof.

13. The method of claim 12, further comprising determining whether said test substance inhibits the biological activity of said protein or its receptor.

14. The method of claim 13, wherein said step of determining whether said test substance inhibits the biological activity of said protein or its receptor comprises a cell-based assay.

15. A kit for the diagnosis of NASH in a patient comprising at least one
a) polynucleotide probe that specifically binds to a polynucleotide encoding a NASH-related protein, or
b) antibody or antigen binding fragment of an antibody that specifically binds to a NASH-related polypeptide,
and instructions for use, wherein said NASH-related protein is the small inducible cytokine subfamily A (Cys-Cys), member 20 (SCYA20) (CCL20).
